(19) Europäisches Patentamt European Patent Office Office européen des brevets

(11) **EP 4 628 883 A1**

(12) **EUROPEAN PATENT APPLICATION**

(43) Date of publication:
08.10.2025 Bulletin 2025/41

(21) Application number: 25168143.3

(22) Date of filing: 03.04.2025

(51) International Patent Classification (IPC):
*G01N 27/22* (2006.01)   *F01M 11/12* (2006.01)
*G01F 23/263* (2022.01)   *G01N 33/28* (2006.01)

(52) Cooperative Patent Classification (CPC):
G01N 27/226; F01M 11/12; G01F 23/265;
G01F 23/268; G01N 33/2888

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 03.04.2024   KR 20240045162
24.07.2024   KR 20240097754
10.09.2024   KR 20240123069
01.04.2025   KR 20250042382

(71) Applicant: **LG Electronics Inc.**
**Yeongdeungpo-gu**
**Seoul 07336 (KR)**

(72) Inventors:
• **KIM, Mangeun**
**Seoul (KR)**
• **YI, Sunghyun**
**Seoul (KR)**
• **PARK, Hyunjin**
**Seoul (KR)**

(74) Representative: **Vossius & Partner**
**Patentanwälte Rechtsanwälte mbB**
**Siebertstrasse 3**
**81675 München (DE)**

(54) **SENSOR**

(57)    The present disclosure relates to a sensor. The sensor of the present disclosure includes an upper electrode extending in a horizontal direction; at least one lower electrode disposed below the upper electrode; and a shield electrode disposed between the upper electrode and the lower electrode, wherein the shield electrode and the lower electrode are disposed to be immersed in a fluid at least containing oil, and a sensing voltage is applied to the upper electrode and the lower electrode.

## FIG. 4A

EP 4 628 883 A1

**Description**

This application claims the priority benefit of Korean Provisional Application No. 10-2024-0045162, filed on April 3, 2024, Korean Provisional Application No. 10-2024-0097754, filed on July 24, 2024, Korean Provisional Application No. 10-2024-0123069, filed on September 10, 2024, and International Application No. Korean Patent Application No. 10-2025-0042382, filed on April 1, 2025.

**BACKGROUND OF THE INVENTION**

**Field of the invention**

This disclosure relates to a sensor, and more particularly, to a sensor for calculating the amount of oil existing inside a compressor.

**Description of the Related Art**

A compressor is installed in a home appliance such as a refrigerator and an air conditioner or in a vehicle to compress refrigerant. The compressor is connected to a condenser and an evaporator, and can compress a refrigerant evaporated from the evaporator and supply it to the condenser.

In order to protect the compressor from mechanical friction, lubrication or cooling is performed through oil, and the compressor must always have a certain level of oil or more. The oil inside the compressor circulates through a refrigerant cycle together with the refrigerant discharged from the compressor. At this time, if the oil accumulates in the condenser, evaporator, and pipe of the refrigerant cycle, it will cause a decrease in the system's capacity, and if the oil recovery is not smooth, the amount of oil inside the compressor will be insufficient, which may cause damage to the compressor. In order to prevent such damage to the compressor, the system performs an oil recovery operation to recover the oil accumulated in the condenser, evaporator, and pipe to the compressor.

In the conventional case, a separate oil level sensor is disposed in the compressor, and oil recovery operation is performed according to the amount of oil detected by the oil level sensor. If an oil level sensor is used, unnecessary oil recovery operation can be reduced, thereby increasing energy efficiency and reliability of compressor performance.

However, according to a conventional method, the oil level sensor is disposed at a certain location inside the compressor, so that it is only possible to check whether the amount of oil existing inside the compressor corresponds to the certain location where the oil level sensor is installed. Thus, there is a problem that a plurality of oil level sensors must be installed at various locations inside the compressor in order to accurately detect the amount of oil existing inside the compressor.

**SUMMARY OF THE INVENTION**

The present disclosure aims to solve the aforementioned problems and other problems.

The disclosure may further provide a sensor capable of accurately detecting the oil concentration inside a compressor.

The disclosure may further provide a sensor capable of accurately detecting the height of the oil surface based on the oil concentration inside a compressor.

The disclosure may further provide a sensor capable of accurately detecting the amount of oil existing inside a compressor.

The disclosure may further provide a sensor optimized to detect the oil concentration, the height of the oil surface and the amount of oil using a plurality of electrodes.

The disclosure may further provide a sensor capable of detecting the height of the oil surface using the self-capacitance method, without the detection range being limited by the size of the electrodes.

The disclosure may further provide various examples of sensing modules of a sensor.

In accordance with an aspect of the present disclosure, a sensor includes an upper electrode extending in a horizontal direction; at least one lower electrode disposed below the upper electrode; and a shield electrode disposed between the upper electrode and the lower electrode, in which the shield electrode and the lower electrode are disposed to be immersed in a fluid at least containing oil, and a sensing voltage is applied to the upper electrode and the lower electrode.

**BRIEF DESCRIPTION OF THE DRAWINGS**

FIG. 1A and FIG. 1B are diagrams illustrating a configuration of a system, according to an embodiment of the present disclosure;
FIG. 2 is a block diagram of a system, according to an embodiment of the present disclosure;
FIG. 3 is a block diagram of a sensor, according to an embodiment of the present disclosure;
FIG. 4A to FIG. 6B are diagrams illustrating a configuration of an electrode assembly, according to embodiments of the present disclosure;
FIG. 7 to FIG. 9 are diagrams for explaining a sensor, according to an embodiment of the present disclosure;
FIG. 10 to FIG. 17 are diagrams illustrating a configuration of an electrode assembly, according to embodiments of the present disclosure;
FIG. 18 to FIG. 23 are diagrams illustrating a sensing module of a sensor, according to an embodiment of the present disclosure;
FIG. 24 to FIG. 27 are diagrams illustrating a sensing module of a sensor, according to an embodiment of the present disclosure; and
FIG. 28 to FIG. 31 are diagrams illustrating a sensing module of a sensor, according to an embodiment of the present disclosure.

## DETAILED DESCRIPTION

Hereinafter, the present disclosure will be described in detail with reference to the drawings. In the drawings, in order to clearly and concisely describe the present disclosure, parts that are not related to the description are omitted, and the same drawing reference numerals are used for identical or extremely similar parts throughout the specification.

The suffixes "module" and "part" used for components in the following description are given simply for the convenience of writing this specification, and do not in themselves impart any particularly important meaning or role. Therefore, the above "module" and "part" may be used interchangeably.

In the present application, it should be understood that the terms "comprises, includes," "has," etc. specify the presence of features, numbers, steps, operations, elements, components, or combinations thereof described in the specification, but do not preclude the presence or addition of one or more other features, numbers, steps, operations, elements, components, or combinations thereof.

In addition, in this specification, terms such as first, second, etc. may be used to describe various elements, but these elements are not limited by these terms. These terms are used only to distinguish one element from another.

Hereinafter, direction is defined based on a rectangular coordinate system. In the rectangular coordinate system, the x-axis direction may be defined as a left-right direction. At this time, based on the origin, the direction toward +x may mean a right direction, and the direction toward -x may mean a left direction. In addition, the y-axis direction may be defined as a front-rear direction. At this time, based on the origin, the direction toward +y may mean a forward direction, and the direction toward -y may mean a rearward direction. In addition, the z-axis direction may be defined as an up-down direction. At this time, based on the origin, the direction toward +z may mean an upward direction, and the direction toward -z may mean a downward direction.

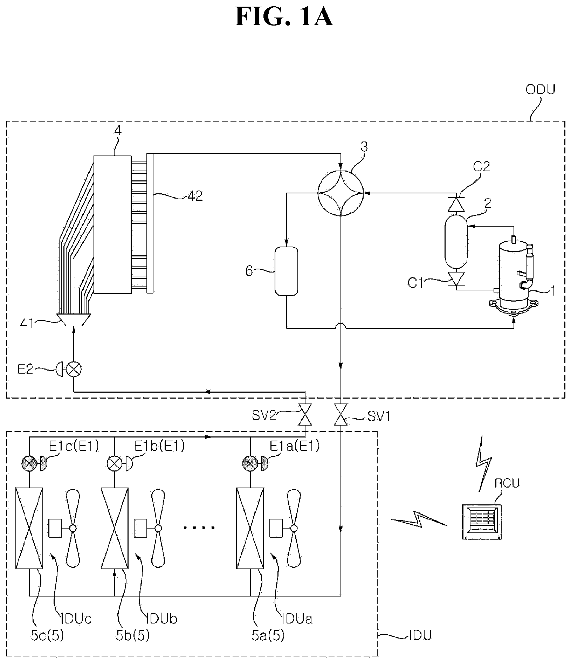

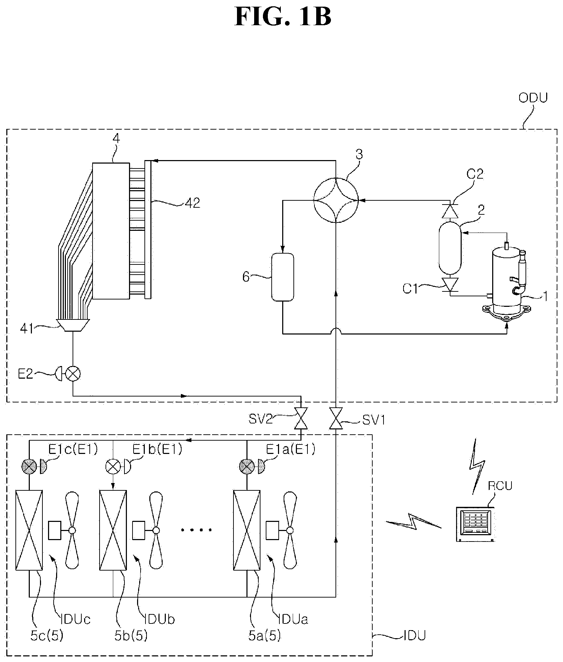

FIG. 1A and FIG. 1B are drawings illustrating a system, according to various embodiments of the present disclosure.

Referring to FIG. 1A and FIG. 1B, the system may include a compressor 1 that compresses a refrigerant. The refrigerant compressed in the compressor 1 may circulate a refrigerant cycle. In this disclosure, it is described that the system is an air conditioner that provides heat-exchanged air to a room based on a refrigerant cycle.

The system may have an outdoor unit (ODU) and an indoor unit (IDU) that are connected to each other by a refrigerant pipe. The system may further have a remote control unit (RCU). The outdoor unit (ODU), the indoor unit (IDU), and/or the remote control unit (RCU) may transmit and receive signals to and from each other.

The outdoor unit (ODU) may have a compressor 1, an oil separator 2, a switching valve 3, an outdoor heat exchanger 4, an outdoor expansion valve E2, and/or an accumulator 6. The indoor unit (IDU) may have an indoor heat exchanger 5 and an indoor expansion valve E1.

The compressor 1 may compress refrigerant flowed in from the accumulator 6 by high temperature and high pressure. For example, the compressor 1 may be an inverter compressor that can control the amount of refrigerant and the discharge pressure of the refrigerant by adjusting an operating frequency. For example, the compressor 1 may be an oil compressor that uses oil as a lubricant.

The oil separator 2 may recover oil from the refrigerant discharged from the compressor 1 and provide it back to the compressor 1. At this time, a first check valve C1 may be installed in a pipe through which the oil separated from the oil separator 2 flows, so as to limit the flow direction of the oil to a direction from the oil separator 2 to the compressor 1.

The switching valve 3 may selectively guide the refrigerant flowed in from the oil separator 2 to the outdoor heat exchanger 4 or the indoor heat exchanger 5. For example, the switching valve 3 may be a four-way valve. At this time, the second check valve C2 may limit the flow direction of the refrigerant to a direction from the oil separator 2 to the switching valve 3.

The outdoor heat exchanger 4 may exchange heat between the refrigerant and the outdoor air. The direction of heat transmission between the refrigerant and the outdoor air in the outdoor heat exchanger 4 may vary depending on the operating mode of the system, i.e., whether it is a heating operation or a cooling operation. An outdoor fan (not shown) is installed in one side of the outdoor heat exchanger 4 and may control the amount of air provided to the outdoor heat exchanger 4.

The indoor heat exchanger 5 may exchange heat between the refrigerant and the indoor air. The direction of heat transmission between the refrigerant and the indoor air in the indoor heat exchanger 5 may vary depending on the operating mode of the system, i.e., whether it is a heating operation or a cooling operation. An indoor fan (not shown) may be installed in one side of the indoor heat exchanger 5 and may control the amount of air provided to the indoor heat exchanger 5.

For example, the indoor heat exchanger 5 may include a plurality of indoor heat exchangers 5a, 5b, 5c. In this case, the indoor unit (IDU) may include a first indoor unit (IDUa) having a first indoor heat exchanger 5a, a first indoor fan, and a first indoor expansion valve E1a, a second indoor unit (IDUb) having a second indoor heat exchanger 5b, a second indoor fan, and a second indoor expansion valve E1b, and a third indoor unit (IDUc) having a third indoor heat exchanger 5c, a third indoor fan, and a third indoor expansion valve Elc. Meanwhile, in response to a required load for indoor cooling or heating, some of a plurality indoor heat exchangers 5a, 5b, 5c may be operated, and the rest may not be operated.

The expansion valve E1, E2 may be installed between the outdoor heat exchanger 4 and the indoor heat exchanger 5, and may expand the refrigerant that has passed through the outdoor heat exchanger 4 or the indoor heat exchanger 5. In addition, the expansion valves E1, E2 may include an outdoor expansion valve E2 adjacent to the outdoor heat exchanger 4 and an indoor expansion valve E1 adjacent to the indoor heat exchanger 5. In this case, the outdoor expansion valve E2 may be used to expand the refrigerant that has passed through the indoor heat exchanger 5, and the indoor expansion valve E1 may be used to expand the refrigerant that has passed through the outdoor heat exchanger 4. For example, the expansion valve E1, E2 may be Electronic Expansion Valve (EEV) capable of controlling the opening degree of the flow path of the refrigerant pipe in which the expansion valve E1, E2 is installed.

For example, the indoor expansion valve E1 may include a first indoor expansion valve Ela that expands the refrigerant provided to the first indoor heat exchanger 5a, a second indoor expansion valve E1b that expands the refrigerant provided to the second indoor heat exchanger 5b, and a third indoor expansion valve Elc that expands the refrigerant provided to the third indoor heat exchanger 5c.

A plurality of sensors (not shown) may measure the temperature and/or pressure of the refrigerant flowing through the refrigerant pipe.

A controller (not shown) may be electrically connected to each component of the system, and may control the operation of each component of the system.

Referring to FIG. 1A, when a heating operation signal is input to the system, the controller may perform heating operation of the system. For example, the heating operation signal may be a signal arbitrarily input by a user. For another example, the heating operation signal may be a signal provided by a thermostat installed in an indoor space to a controller, when the indoor temperature detected by the indoor temperature sensor is lower than a desired temperature set by a user by a certain level or higher.

Specifically, the low-temperature, low-pressure refrigerant flowing from the accumulator 6 to the compressor 1 may be compressed by high temperature, high pressure in the compressor 1 and discharged to the oil separator 2. Then, the refrigerant from which oil is separated in the oil separator 2 may flow into the second indoor heat exchanger 5b via the switching valve 3 and the first service valve SV1. At this time, the second indoor expansion valve E1b may completely open the refrigerant flow path that passes through the second indoor heat exchanger 5b and leads to the outdoor heat exchanger 4. In addition, the first indoor expansion valve Ela and the third indoor expansion valve Elc can close the refrigerant flow path that passes through the first indoor heat exchanger 5a and the third indoor heat exchanger 5c and leads to the outdoor heat exchanger 4. In addition, when a required heating load increases, the first indoor expansion valve Ela and/or the third indoor expansion valve Elc may also be opened.

As heat energy is transmitted from the refrigerant to the indoor air in the second indoor heat exchanger 5b, the refrigerant may be condensed. At this time, the second indoor heat exchanger 5b may serve as a condenser. In addition, as the heat exchange occurs between the refrigerant and the indoor air, the indoor space may be heated. The refrigerant condensed while passing through the second indoor heat exchanger 5b may pass through the outdoor expansion valve E2, via the second indoor expansion valve E1b and a second service valve SV2. The refrigerant expanded while passing through the outdoor expansion valve E2 may be distributed to multiple points of the outdoor heat exchanger 4 via a distributor 41.

As the heat energy of the outdoor air is transmitted to the refrigerant in the outdoor heat exchanger 4, the refrigerant may be evaporated. At this time, the outdoor heat exchanger 4 may serve as an evaporator. The refrigerant evaporated while passing through the outdoor heat exchanger 4 may be flowed into the compressor 1 via the header 42, the switching valve 3, and the accumulator 6 sequentially. Thus, a refrigerant cycle for the heating operation of the aforementioned system can be completed.

Referring to FIG. 1B, when a cooling operation signal is input to the system, the controller may perform the cooling operation of the system. For example, the cooling operation signal may be a signal arbitrarily input by a user. For another example, the cooling operation signal may be a signal provided by a thermostat installed in the indoor space to the controller, when the indoor temperature detected by the indoor temperature sensor is higher than a desired temperature set by a user by a certain level or higher.

Specifically, the low-temperature, low-pressure refrigerant flowing from the accumulator 6 to the compressor 1 may be compressed by a high temperature, high pressure in the compressor 1 and discharged to the oil separator 2. Then, the refrigerant from which oil is separated in the oil separator 2 may be flowed into the outdoor heat exchanger 4 via the switching valve 3 and the header 42.

As heat energy is transmitted from the refrigerant to the outdoor air in the outdoor heat exchanger 4, the refrigerant may be condensed. At this time, the outdoor heat exchanger 4 may serve as a condenser.

The refrigerant condensed while passing through the outdoor heat exchanger 4 may flow into the second indoor expansion valve E1b via the distributor 41, the outdoor expansion valve E2, and the second service valve SV2 sequentially. At this time, the outdoor expansion valve E2 may completely open the flow path. Then, the refrigerant expanded while passing through the second indoor expansion valve E1b may flow into the second indoor heat exchanger 5b. In addition, when the required cooling load increases, the first indoor expansion valve Ela and/or the third indoor expansion valve Elc may also be opened by a certain degree.

As the heat energy of the indoor air is transmitted to the refrigerant in the second indoor heat exchanger 5b, the refrigerant may evaporate. At this time, the second indoor heat exchanger 5b may serve as an evaporator. Then, the indoor space may be cooled by the heat exchange between the refrigerant and the indoor air. The refrigerant that has evaporated while passing through the second indoor heat exchanger 5b may be flowed into the compressor 1 via the first service valve SV1, the switching valve 3, and the accumulator 6 sequentially. Thus, a refrigerant cycle for cooling operation of the aforementioned system may be completed.

Meanwhile, the system may perform an oil recovery operation. For example, when the system performs an oil recovery operation, the system may control the switching valve 3 so that the refrigerant discharged from the compressor 1 flows to the outdoor heat exchanger 4. At this time, the refrigerant may be condensed by heat exchange between the refrigerant and the outdoor air that occurs in the outdoor heat exchanger 4. The refrigerant condensed in the outdoor heat exchanger 4 may be flowed into the indoor unit (IDU). The refrigerant may be evaporated by heat exchange between the refrigerant flowed into the indoor unit (IDU) and the indoor air.

According to an embodiment, when performing an oil recovery operation, the system may increase the operating frequency of the compressor 1 to a certain level or more. For example, when performing an oil recovery operation, the system may set the operating frequency of the compressor 1 to the maximum value. When performing an oil recovery operation, the system may stop the operation of an indoor fan. When performing an oil recovery operation, the system may control the operation of the outdoor fan, based on the pressure of the outdoor unit-side pipe. For example, if the pressure of the outdoor unit-side pipe exceeds a certain standard, the outdoor fan may be operated, and if it is below the certain standard, the operation of the outdoor fan may be stopped.

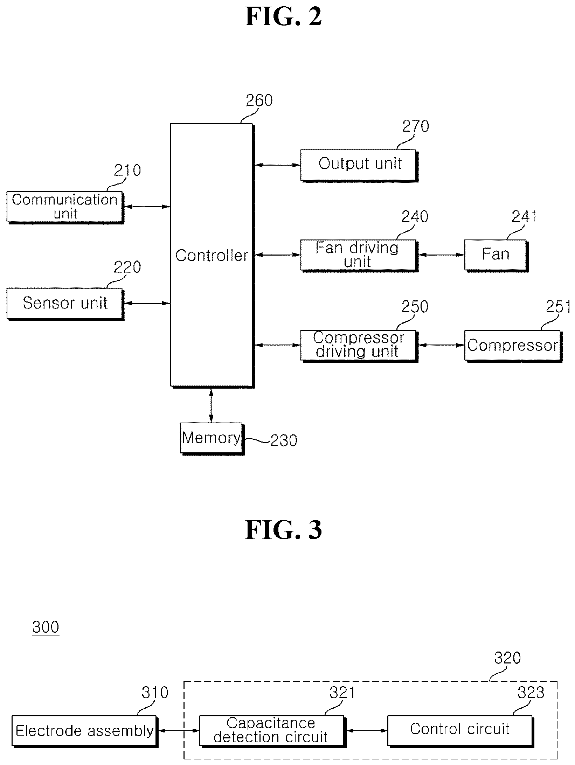

FIG. 2 is a block diagram of a system, according to an embodiment of the present disclosure.

Referring to FIG. 2, the system may include a communication unit 210, a sensor unit 220, a memory 230, a fan driving unit 240 that drives a fan 241, a compressor driving unit 250 that drives a compressor 251 (compressor 1 of FIG. 1A), and/or a controller 260.

The communication unit 210 may include at least one communication module. For example, the communication unit 210 may be provided in each of the outdoor unit (ODU) and the indoor unit (IDU), and the outdoor unit (ODU) and the indoor unit (IDU) may transmit and receive data to and from each other. For example, the communication unit 210 may be provided in the remote control unit (RCU).

The communication method of the outdoor unit (ODU), the indoor unit (IDU), and/or the remote control unit (RCU) may be, for example, a communication method using a power line, a serial communication method (e.g., RS-485 communication), a wired communication method through refrigerant piping, or a wireless communication method such as Wi-fi, Bluetooth, Beacon, and Zigbee.

The communication unit 210 may transmit and receive data to and from an external device. For example, the communication unit 210 may access a server connected to an external network to transmit and receive data.

The sensor unit 220 may have at least one sensor, and may transmit data on a detection value detected through the sensor to the controller 260.

The sensor unit 220 may have a heat exchanger temperature sensor (not shown). For example, the heat exchanger temperature sensor may be arranged inside the indoor heat exchanger 5, and may detect the temperature of the indoor heat exchanger 5.

The sensor unit 220 may have a pipe temperature sensor (not shown). The pipe temperature sensor may detect the temperature of the refrigerant flowing through each pipe of the system. For example, the pipe temperature sensor may be arranged in the inlet pipe of the indoor unit (IDU) and/or the outlet pipe of the indoor unit (IDU), and may detect the temperature of the refrigerant flowing through the pipe. For example, the pipe temperature sensor may be arranged in a pipe connected to the compressor 251 to detect the temperature (hereinafter, suction temperature) of the refrigerant flowing into the compressor 251 and/or the temperature (hereinafter, discharge temperature) of the refrigerant discharged from the compressor 251.

The sensor unit 210 may have a pressure sensor (not shown). The pressure sensor (not shown) may detect the pressure of the gaseous refrigerant flowing through each pipe of the system. For example, the pressure sensor may be arranged in a pipe connected to the compressor 251 to detect the pressure (hereinafter, suction pressure) of the refrigerant flowing into the compressor 251 and/or the pressure (hereinafter, discharge pressure) of the refrigerant discharged from the compressor 251.

The sensor unit 220 may have an indoor temperature sensor (not shown) that detects the indoor temperature and/or an outdoor temperature sensor (not shown) that detects the outdoor temperature.

The sensor unit 220 may have an indoor humidity sensor (not shown) that detects indoor humidity and/or an outdoor humidity sensor (not shown) that detects outdoor humidity.

The sensor unit 220 may include a sensor 300 (see FIG. 3) (hereinafter, oil sensor) that outputs a signal corresponding to oil stored inside the compressor 251. The oil sensor 300 may detect the height of the fluid surface inside the compressor 251. The oil sensor 300 may detect the amount of the fluid stored inside the compressor 251. The oil sensor 300 may detect the oil concentration with respect to the fluid. The fluid stored inside the compressor 251 may at least include oil. The fluid stored inside the compressor 251 may further include a refrigerant.

The memory 230 may store data related to the operation of each component provided in the system.

The memory 230 may store programs for processing and controlling each signal within the controller 260, and may store processed data and data to be processed. For example, the memory 230 may store application programs designed for the purpose of performing various tasks that can be processed by the controller 260, and may selectively provide some of the stored application programs when requested by the controller 260.

The memory 230 may include, for example, at least one of volatile memory (e.g., DRAM, SRAM, SDRAM, etc.) and nonvolatile memory (e.g., flash memory, hard disk drive (HDD), solid-state drive (SSD), etc.).

The fan driving unit 240 may drive the fan 241 provided in the system. For example, the fan 241 may include an outdoor fan and/or an indoor fan.

The fan driving unit 240 may include a rectifier (not shown) that rectifies AC power into DC power and outputs it, a DC-link capacitor (not shown) that stores a pulsating voltage from the rectifier, an inverter (not shown) that has a plurality of switching elements and converts and outputs smoothed DC power into three-phase AC power of a certain frequency, and/or at least one motor that drives the fan 241 according to the three-phase AC power output from the inverter.

Meanwhile, the fan driving unit 240 may be provided with separate configurations for driving the outdoor fan and the indoor fan, respectively. For example, the system may include a first fan driving unit for driving the outdoor fan and a second fan driving unit for driving the indoor fan.

The compressor driving unit 250 may drive the compressor 251. The compressor driving unit 250 may include a rectifier (not shown) that rectifies AC power into DC power and outputs it, a DC-link capacitor (not shown) that stores a pulsating voltage from the rectifier, an inverter (not shown) that has a plurality of switching elements, and converts and outputs smoothed DC power into three-phase AC power of a certain frequency, and/or a compressor motor 102b that drives the compressor 251 according to the three-phase AC power output from the inverter.

The controller 260 may control the overall operation of the system. The controller 260 may be connected to each component provided in the system, and may control the overall operation of each component by transmitting and/or receiving signals to/and from each component.

The controller 260 may control the operation of the fan driving unit 240 and change the rotation speed of the fan 241. For example, the fan driving unit 240 may change the rotation speed of the outdoor fan by changing the frequency of the three-phase AC power output to the outdoor fan motor, according to the control of the controller 260. For example, the fan driving unit 240 may change the rotation speed of the indoor fan by changing the frequency of the three-phase AC power output to the indoor fan motor, according to the control of the controller 260.

The controller 260 may control the operation of the compressor driving unit 250 to change the operating frequency of the compressor 251. For example, the compressor driving unit 250 may change the operating frequency of the compressor 251, by changing the frequency of the three-phase AC power output to the compressor motor 102b, according to the control of the controller 260.

The controller 260 may be equipped not only in the outdoor unit (ODU), but also in the indoor unit (IDU), the outdoor unit (ODU), and/or the remote control unit (RCU).

The controller 260 may include at least one processor, and may control the overall operation of the system by using the processor included therein. Here, the processor may be a general processor such as a central processing unit (CPU). Obviously, the processor may be a dedicated device such as an ASIC or another hardware-based processor.

The controller 260 may obtain data related to each component provided in the system. At this time, the controller 260 may obtain data related to each component provided in the system at regular time intervals, according to a certain cycle, in consideration of a computational load.

The controller 260 may perform various computations based on the acquired data, and may control the overall operation of each component provided in the system according to a computational result.

Data related to each component provided in the system may include, for example, the operating frequency of the compressor 251, the suction temperature of the compressor 251, the discharge temperature, the suction pressure, the discharge pressure, the oil concentration stored inside the compressor 251, the amount of oil, the inlet-side pipe temperature of the indoor unit (IDU), the outlet-side pipe temperature of the indoor unit (IDU), the indoor temperature, the outdoor temperature, the opening amount of the electronic expansion valve (EEV), etc.

Meanwhile, the system may further include an input device (not shown) capable of receiving an user input. For example, when the system receives a user input through an input device (e.g., a touch panel, a key, etc.,) the system may perform an operation corresponding to the received user input.

The system may further include an output device 270 that outputs a message related to the operating state of the system. For example, the output device 270 may include a display device such as a display and a light emitting diode LED and/or an audio device such as a speaker and a buzzer.

FIG. 3 is a block diagram of a sensor, according to an embodiment of the present disclosure.

Referring to FIG. 3, the oil sensor 300 may include an electrode assembly 310 and/or a processing circuit 320.

The electrode assembly 310 may include a plurality of electrodes. Some of the plurality of electrodes included in the electrode assembly 310 may be formed to extend vertically. Others of the plurality of electrodes included in the electrode assembly 310 may be formed to extend horizontally.

The electrode assembly 310 may be dispos3ed inside the compressor 1. The electrode assembly 310 may be exposed to a fluid stored inside the compressor 1. At least some of the plurality of electrodes included in the electrode assembly 310 may be arranged to be immersed in the fluid. The fluid stored inside the compressor 1 may at least include oil. The fluid stored inside the compressor 1 may further include a refrigerant.

A voltage may be applied to the electrode assembly 310. A sensing voltage may be applied to some of the plurality of electrodes included in the electrode assembly 310. The sensing voltage may be an AC voltage whose voltage value changes periodically. The sensing voltage may have a certain frequency. A ground voltage may be applied to others of the plurality of electrodes included in the electrode assembly 310.

The processing circuit 320 may be electrically connected to the electrode assembly 310. The processing circuit 320 may apply a voltage to the electrode assembly 310.

The processing circuit 320 may detect capacitance, based on the voltage applied to the electrode assembly 310.

For example, the processing circuit 320 may detect, by using a mutual-capacitance method, capacitance corresponding to a first electrode to which a sensing voltage is applied and a second electrode to which a ground voltage is applied among a plurality of electrodes included in the electrode assembly 310. The capacitance corresponding to two electrodes among the plurality of electrodes may be detected based on Equation 1.

[Equation 1]

$$C = \varepsilon_0 \varepsilon_r \frac{A}{d}$$

Here, C may mean capacitance, $\varepsilon 0$ may mean permittivity corresponding to vacuum, $\varepsilon r$ may mean relative permittivity corresponding to a material between two electrodes, d may mean a distance between two electrodes, and A may mean an area of an electrode in contact with a material.

For example, the processing circuit 320 may detect the capacitance corresponding to a single electrode to which the sensing voltage is applied among the plurality of electrodes included in the electrode assembly 310. At this time, the processing circuit 320 may detect the capacitance corresponding to a single electrode to which the sensing voltage is applied through a self-capacitance method.

The processing circuit 320 may calculate the oil concentration contained in the fluid stored inside the compressor 1, based on the capacitance for the electrode assembly 310. The processing circuit 320 may calculate the height of the fluid surface inside the compressor 1, based on the capacitance for the electrode assembly 310. The processing circuit 320 may calculate the amount of fluid stored inside the compressor 1, based on the capacitance for the electrode assembly 310.

The processing circuit 320 may include a capacitance detection circuit 321 that detects the capacitance for the electrode assembly 310. The processing circuit 320 may include a control circuit 323 that calculates the oil concentration, the height of the fluid surface and/or the amount of fluid. The capacitance detection circuit 321 and the control circuit 323 may be implemented as one configuration or may be implemented as separate configurations. Meanwhile, the capacitance detection circuit 321 and/or the control circuit 323 may be included in the controller 260 of the system.

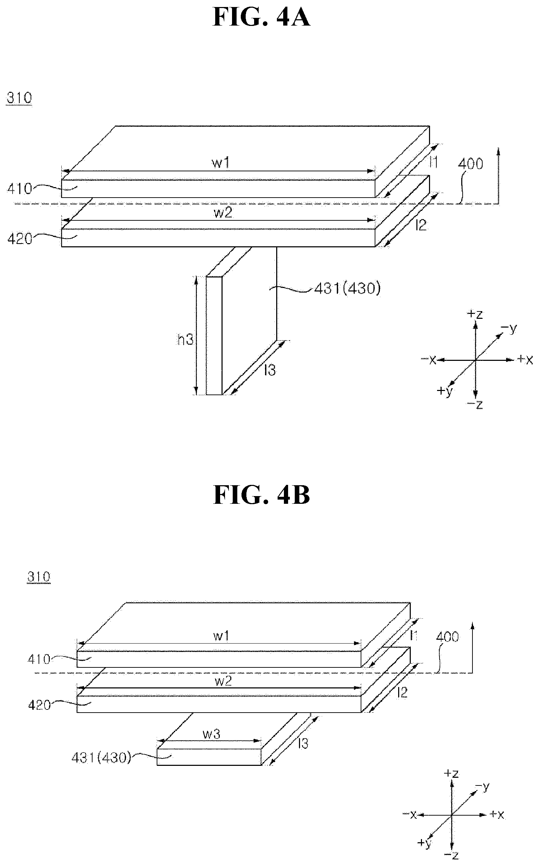

FIG. 4A to FIG. 6B are diagrams illustrating a configuration of an electrode assembly, according to embodiments of the present disclosure.

Referring to FIG. 4A to FIG. 6B, the electrode assembly 310 may include an upper electrode 410, a shield electrode 420, and a lower electrode assembly 430.

The upper electrode 410 may be formed to extend horizontally. The upper electrode 410 may be formed to have a first width w1 along the left-right direction. The upper electrode 410 may be formed to have a first length l1 along the front-rear direction. The upper electrode 410 may be formed to have a first height along the up-down direction.

The upper electrode 410 may be disposed so that the upper surface having the first width w1 and the first length l1 faces upward. The upper electrode 410 may be disposed so that the lower surface having the first width w1 and the first length l1 directs downward.

A sensing voltage may be applied to the upper electrode 410. The capacitance corresponding to the upper electrode 410 may be detected by using a self-capacitance method. When the height of the surface 400 of the fluid changes, the amount of fluid existing between the upper plane of the upper electrode 410 and the fluid surface 400 may change. Here, the height of the fluid surface 400 may correspond to a distance between the upper electrode 410 and the fluid surface 400.

At this time, the capacitance corresponding to the upper electrode 410 may change in response to the change in the amount of fluid existing between the upper plane of the upper electrode 410 and the fluid surface 400. That is, the capacitance corresponding to the upper electrode 410 may correspond to the height of the fluid surface 400. In addition, the capacitance corresponding to the upper electrode 410 may correspond to the amount of fluid stored inside the compressor 251.

The capacitance corresponding to the upper electrode 410 may correspond to the concentration of oil contained in the fluid existing between the upper electrode 410 and the fluid surface 400. For example, as the concentration of oil becomes higher, the change in the capacitance corresponding to the upper electrode 410 according to the height of the fluid surface 400 may become smaller. Therefore, since the capacitance corresponding to the upper electrode 410 varies depending on the concentration of oil, the control circuit 323 may first determine the concentration of oil, and then determine the height of the fluid surface 400 based on the concentration of oil. In addition, the control circuit 323 may determine the amount of fluid in response to a determination of the height of the fluid surface 400.

The shield electrode 420 may be formed to extend horizontally.

The shield electrode 420 may be formed to have a second width w2 along the left-right direction. The second width w2 of the shield electrode 420 may correspond to the first width w1 of the upper electrode 410. For example, the second width w2 of the shield electrode 420 may be greater than or equal to the first width w1 of the upper electrode 410.

The shield electrode 420 may be formed to have a second length l2 along the front-rear direction. The second length l2 of the shield electrode 420 may correspond to the first length l1 of the upper electrode 410. For example, the second length l2 of the shield electrode 420 may be greater than or equal to the first length l1 of the upper electrode 410.

The shield electrode 420 may be formed to have a second height along the up-down direction.

The shield electrode 420 may be disposed below the upper electrode 410. The shield electrode 420 may be disposed to correspond to the upper electrode 410. In the horizontal direction, the size of the upper electrode 410 may be smaller than the size of the shield electrode 420. For example, when viewing the upper surface of the upper electrode 410 in the downward direction, the upper surface of the upper electrode 410 may be located inside the shield electrode 420.

The shield electrode 420 may be disposed so that at least a part of the shield electrode 420 is immersed in the fluid stored inside the compressor 1. In the case where a minimum amount of fluid is stored inside the compressor 1, the fluid surface 400 may be located above the lower surface of the shield electrode 420. That is, the shield electrode 420 may be disposed so that the lower surface of the shield electrode 420 is immersed in the fluid stored inside the compressor 1.

The lower electrode assembly 430 may be disposed below the shield electrode 420. As the lower electrode assembly 430 is disposed on the lower side of the shield electrode 420, the lower electrode assembly 430 may be disposed to be immersed in the fluid stored inside the compressor 1. That is, in the case where a minimum amount of fluid is stored inside the compressor 1, the fluid surface 400 may be located above the lower electrode assembly 430.

The lower electrode assembly 430 may be disposed to correspond to the shield electrode 420. The shield electrode 420 may be disposed to cover the lower electrode assembly 430 from above. That is, in the horizontal direction, the size of the lower electrode assembly 430 may be smaller than the size of the shield electrode 420. For example, when viewing the lower surface of the shield electrode 420 in the upper direction, the lower electrode assembly 430 may be located inside the shield electrode 420.

As the shield electrode 420 is disposed on the upper side of the lower electrode assembly 430 to cover the lower electrode assembly 430, the influence of the upper electrode 410 on the lower electrode assembly 430 may be reduced. In addition, as the shield electrode 420 is disposed on the upper side of the lower electrode assembly 430 to cover the lower electrode assembly 430, the influence of the change in the height of the fluid surface 400 on the lower electrode assembly 430 may be reduced.

The lower electrode assembly 430 may include at least one lower electrode to which a sensing voltage is applied. The lower electrode assembly 430 may further include a ground electrode 433 to which a ground voltage is applied.

Referring to FIGS. 4A and 4B, the lower electrode assembly 430 can include a first lower electrode 431.

As shown in FIG. 4A, the first lower electrode 431 may be formed to extend vertically. The first lower electrode 431 may be formed to have a third height h3 along the up-down direction. The first lower electrode 431 may be formed to have a third length l3 along the front-rear direction.

Meanwhile, as shown in FIG. 4B, the first lower electrode 431 may be formed to extend horizontally. The first lower electrode 431 may be formed to have a third width w3 along the left-right direction. The first lower electrode 431 may be formed to have a third length l3 along the front-rear direction.

The capacitance corresponding to the first lower electrode 431 may be detected using a self-capacitance method. At this time, since the first lower electrode 431 is always immersed in the fluid, the capacitance corresponding to the first lower electrode 431 may correspond to the concentration of oil contained in the fluid. That is, the sensor 300 may determine the concentration of oil contained in the fluid, based on the capacitance corresponding to the first lower electrode 431.

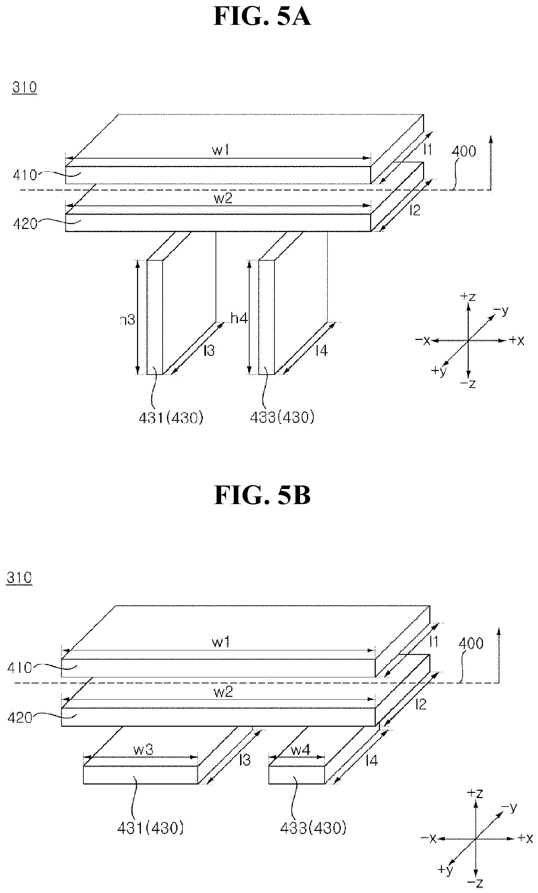

Referring to FIGS. 5A and 5B, the lower electrode assembly 430 may include a ground electrode 433.

As shown in FIG. 5A, the ground electrode 433 may be formed to extend vertically. The ground electrode 433 may be formed to have a fourth height h4 along the up-down direction. The ground electrode 433 may be formed to have a fourth length l4 along the front-rear direction. For example, the fourth height h4 of the ground electrode 433 may correspond to the third length h3 of the first lower electrode 431. For example, the fourth length l4 of the ground electrode 433 may correspond to the third length l3 of the first lower electrode 431.

Meanwhile, as shown in FIG. 5B, the first lower electrode 431 may be formed to extend horizontally. The ground electrode 433 may be formed to have a fourth width w4 along the left-right direction.

The capacitance corresponding to the first lower electrode 431 may be detected by using a mutual capacitance method for the first lower electrode 431 and the ground electrode 433. At this time, since the first lower electrode 431 and the ground electrode 433 are always immersed in the fluid, the capacitance corresponding to the first lower electrode 431 may correspond to the concentration of oil contained in the fluid. That is, the sensor 300 may determine the concentration of oil contained in the fluid, based on the capacitance corresponding to the first lower electrode 431.

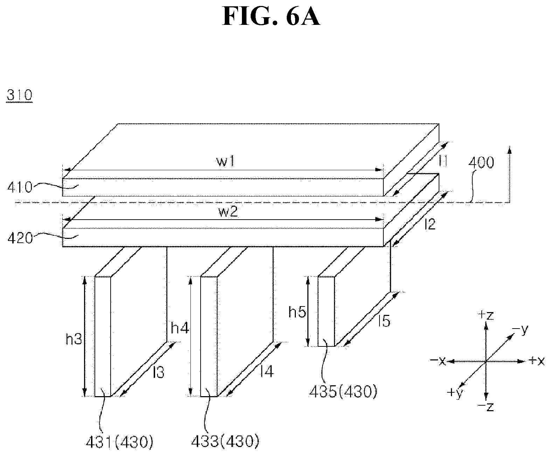

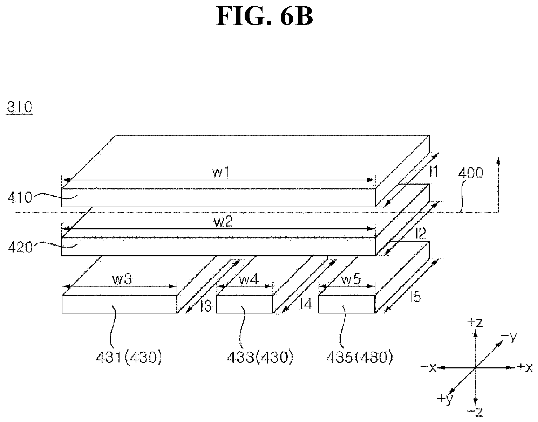

Referring to FIGS. 6A and 6B, the lower electrode assembly 430 may include a second lower electrode 435.

As shown in FIG. 6A, the second lower electrode 435 may be formed to extend vertically. The second lower electrode 435 may be formed to have a fifth height h5 along the up-down direction. The second lower electrode 435 may be formed to have a fifth length l5 along the front-rear direction. For example, the fifth length l5 of the second lower electrode 435 may correspond to the third length l3 of the first lower electrode 431 and the fourth length l4 of the ground electrode 433. For example, the fifth height h5 of the second lower electrode 435 may be different from the third height h3 of the first lower electrode 431.

Meanwhile, as shown in FIG. 6B, the second lower electrode 435 may be formed to be horizontally extended. The second lower electrode 435 may be formed to have a fifth width w5 along the left-right direction. The second lower electrode 435 may be formed to have a fifth length l5 along the front-rear direction. For example, the fifth width w5 of the second lower electrode 435 may be different from the third width w3 of the first lower electrode 431.

The capacitance corresponding to the first lower electrode 431 may be detected by using a mutual capacitance method for the first lower electrode 431 and the ground electrode 433. Since the first lower electrode 431 and the ground electrode 433 are immersed in the fluid, the capacitance corresponding to the first lower electrode 431 and the ground electrode 433 may correspond to the concentration of oil contained in the fluid.

The capacitance corresponding to the second lower electrode 435 may be detected by using the mutual capacitance method for the second lower electrode 435 and the ground electrode 433. Since the second lower electrode 435 and the ground electrode 433 are immersed in the fluid, the capacitance corresponding to the second lower electrode 435 and the ground electrode 433 may correspond to the concentration of oil contained in the fluid.

As shown in FIG. 6A, in the case where the first lower electrode 431 and the second lower electrode 435 are formed to extend vertically, since the third height h3 of the first lower electrode 431 and the fifth height h5 of the second lower electrode 435 are different, the capacitance corresponding to the first lower electrode 431 and the capacitance corresponding to the second lower electrode 435 may be different.

At this time, if the oil concentration for the depth of the fluid directed downward is different depending on the depth of the fluid, the difference in the oil concentration for the depth of the fluid may affect the difference between the capacitance corresponding to the first lower electrode 431 and the capacitance corresponding to the second lower electrode 435.

The capacitance corresponding to the first lower electrode 431 may correspond to the oil concentration for the depth of the fluid corresponding to the third height h3. The capacitance corresponding to the second lower electrode 435 may correspond to the oil concentration for the depth of the fluid corresponding to the fifth height h5.

According to an embodiment, the control circuit 323 may determine the oil concentration corresponding to the depth of the fluid. For example, the control circuit 323 may determine the oil concentration for the depth of the fluid corresponding to the fifth height h5, based on the capacitance corresponding to the second lower electrode 435. At this time, the control circuit 323 may determine the oil concentration for the depth of the fluid corresponding to the third height h3, based on the capacitance corresponding to the first lower electrode 431 and the oil concentration for the depth of the fluid corresponding to the fifth height h5.

Meanwhile, as in FIG. 6B, in the case where the first lower electrode 431 and the second lower electrode 435 are formed to extend horizontally, since the third width w3 of the first lower electrode 431 and the fifth width w5 of the second lower electrode 435 are different, the capacitance corresponding to the first lower electrode 431 and the capacitance corresponding to the second lower electrode 435 may be different.

The capacitance corresponding to the first lower electrode 431 may correspond to the oil concentration for the depth of the fluid corresponding to the third width w3 of the first lower electrode 431. The capacitance corresponding to the second lower electrode 435 may correspond to the oil concentration for the depth of the fluid corresponding to the fifth width w5 of the second lower electrode 435. For example, when the third width w3 is larger than the fifth width w5, the depth of the fluid corresponding to the third width w3 may be larger than the depth of the fluid corresponding to the fifth width w5.

According to an embodiment, the control circuit 323 may determine the oil concentration for the depth of the fluid. For example, the control circuit 323 may determine the oil concentration for the depth of the fluid corresponding to the third width w3, based on the capacitance corresponding to the first lower electrode 431. For example, the control circuit 323 may determine the oil concentration for the depth of the fluid corresponding to the fifth width w5, based on the capacitance corresponding to the second lower electrode 435.

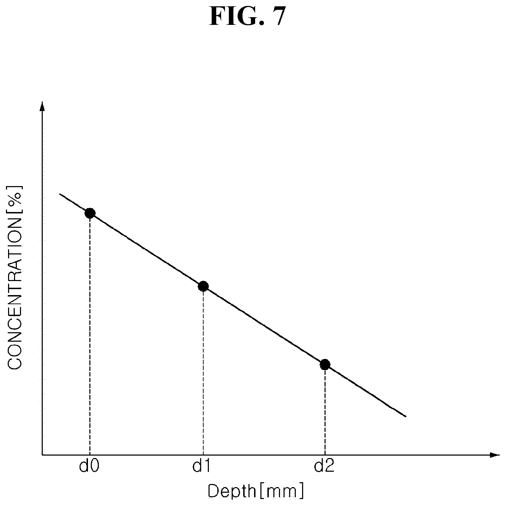

Referring to FIG. 7, when the first concentration of oil corresponding to the first depth d1 of the fluid and the second concentration of oil corresponding to the second depth d2 of the fluid are different from each other, the control circuit 323 may determine the concentration of oil corresponding to the fluid surface d0, based on the first concentration of oil and the second concentration of oil. For example, the control circuit 323 may determine the concentration of oil corresponding to the fluid surface d0, based on the difference between the first concentration and the second concentration for the difference between the first depth d1 and the second depth d2. For example, the control circuit 323 may determine the concentration of oil corresponding to the fluid surface d0, based on data on the concentration of oil corresponding to the fluid surface d0, corresponding to the first concentration, the second concentration, and the difference between the first concentration and the second concentration.

Therefore, according to various embodiments of the present disclosure, the control circuit 323 may first determine the concentration of oil, and then determine the height of the fluid surface based on the concentration of oil. For example, the control circuit 323 may determine the concentration of oil based on the capacitance corresponding to the first lower electrode 431, and then determine the height of the fluid surface based on the determined concentration of oil. For example, the control circuit 323 may determine the concentration of oil based on the capacitance corresponding to the second lower electrode 435, and then determine the height of the fluid surface based on the determined concentration of oil. For example, the control circuit 323 may determine the concentration of oil corresponding to the fluid surface based on the capacitance corresponding to the first lower electrode 431 and the capacitance corresponding to the second lower electrode 435, and then determine the height of the fluid surface based on the determined concentration of oil.

According to an embodiment, a sensing voltage may be applied to the shield electrode 420. That is, the shield electrode 420 may be configured for active shielding.

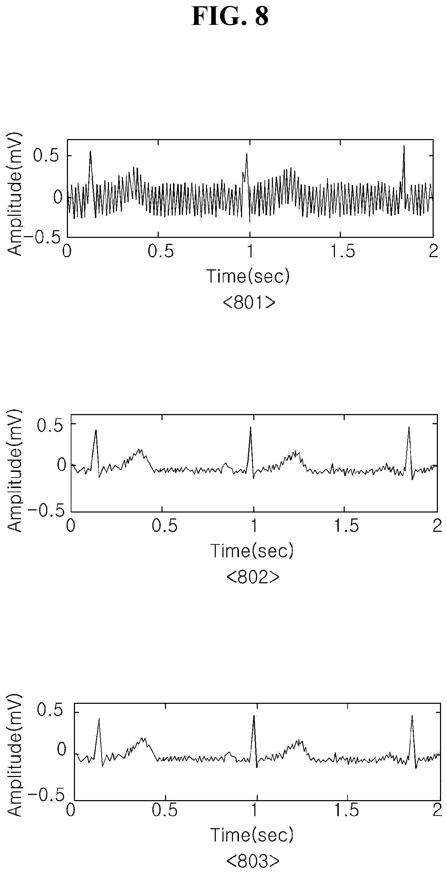

Referring to FIG. 8, when reviewing the output of the sensor in a time domain, if (802) there is a configuration for passive shielding, or if (803) there is a configuration for active shielding, the noise included in the output of the sensor may be significantly reduced, in comparison with a case (801) where there is no configuration for passive shielding or active shielding.

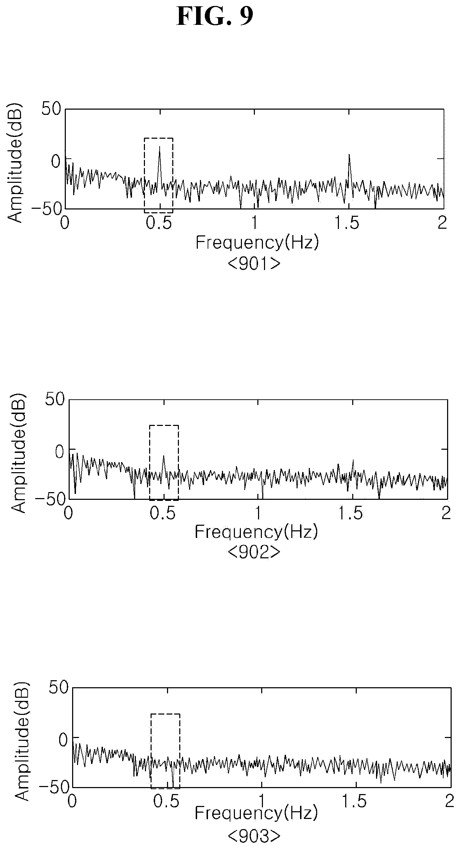

Meanwhile, referring to FIG. 9, when reviewing the output of the sensor in a frequency domain, if (902) there is a configuration for passive shielding, the noise peak at a certain frequency (e.g., 50 Hz) may be reduced, in comparison with a case (801) where there is no configuration for passive shielding or active shielding. In addition, if (903) there is a configuration for active shielding, the noise peak at a certain frequency (e.g., 50 Hz) may be further reduced, in comparison with a case (902) where there is a configuration for passive shielding.

As described above, when a sensing voltage is applied to the shield electrode 420, a potential difference does not occur between the shield electrode 420 and the lower electrode 431, 435, so that the parasitic capacitance may be eliminated. Therefore, due to the elimination of the parasitic capacitance, the signal-to-noise ratio SNR may be improved.

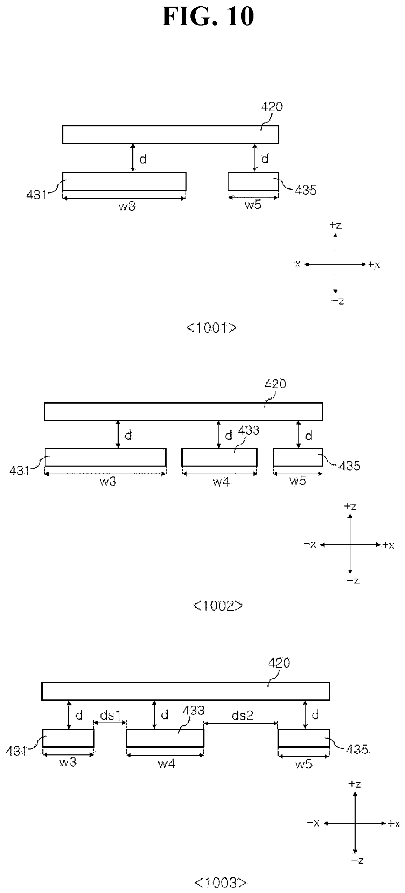

FIG. 10 to FIG. 17 are diagrams illustrating a configuration of an electrode assembly, according to embodiments of the present disclosure.

Referring to FIGS. 10 to 17, depending on the shape, size, position, and distance from other configurations of each of the first lower electrode 431 and the second lower electrode 435 according to embodiments of the present disclosure, the capacitance corresponding to the first lower electrode 431 and the capacitance corresponding to the second lower electrode 435 may be different from each other. At this time, the difference between the capacitance corresponding to the first lower electrode 431 and the capacitance corresponding to the second lower electrode 435 may correspond to the difference in oil concentration for the depth of the fluid directed downward. Accordingly, the sensor 300 may determine the oil concentration corresponding to the depth of the fluid, based on the capacitance corresponding to the first lower electrode 431, the capacitance corresponding to the second lower electrode 435, and/or the difference between the two capacitances.

Referring to FIG. 10, the lower electrode assembly 430 may include the first lower electrode 431 and the second lower electrode 435. The first lower electrode 431 and the second lower electrode 435 may be formed to extend horizontally.

The first lower electrode 431 and the second lower electrode 435 may be disposed to be spaced apart from the shield electrode 420 in the vertical direction by a certain distance d. The first lower electrode 431 and the second lower electrode 435 may be disposed side by side along the horizontal direction.

Referring to the reference numeral 1001 of FIG. 10, the third width w3 of the first lower electrode 431 may be different from the fifth width w5 of the second lower electrode 435.

The capacitance corresponding to the first lower electrode 431 and the capacitance corresponding to each second lower electrode 435 may be detected by using the self-capacitance method. At this time, since the third width w3 of the first lower electrode 431 and the fifth width w5 of the second lower electrode 435 are different, the capacitance corresponding to the first lower electrode 431 and the capacitance corresponding to the second lower electrode 435 may be different.

Referring to reference numeral 1002 of FIG. 10, the third width w3 of the first lower electrode 431 may be different from the fifth width w5 of the second lower electrode 435.

A ground electrode 433 may be disposed between the first lower electrode 431 and the second lower electrode 435.

The capacitance corresponding to the first lower electrode 431 and the capacitance corresponding to each second lower electrode 435 may be detected by using the mutual capacitance method for the ground electrode 433. At this time, since the third width w3 of the first lower electrode 431 and the fifth width w5 of the second lower electrode 435 are different, the capacitance corresponding to the first lower electrode 431 and the capacitance corresponding to the second lower electrode 435 may be different.

Referring to reference numeral 1003 of FIG. 10, the third width w3 of the first lower electrode 431 may correspond to the fifth width w5 of the second lower electrode 435.

The ground electrode 433 may be disposed between the first lower electrode 431 and the second lower electrode 435. In the horizontal direction, the distance ds1 between the first lower electrode 431 and the ground electrode 433 and the distance ds2 between the second lower electrode 435 and the ground electrode 433 may be different.

The capacitance corresponding to the first lower electrode 431 and the capacitance corresponding to each second lower electrode 435 may be detected by using the mutual capacitance method for the ground electrode 433. At this time, since the distance dsl between the first lower electrode 431 and the ground electrode 433 and the distance ds2 between the second lower electrode 435 and the ground electrode 433 are different, the capacitance corresponding to the first lower electrode 431 and the capacitance corresponding to the second lower electrode 435 may be different.

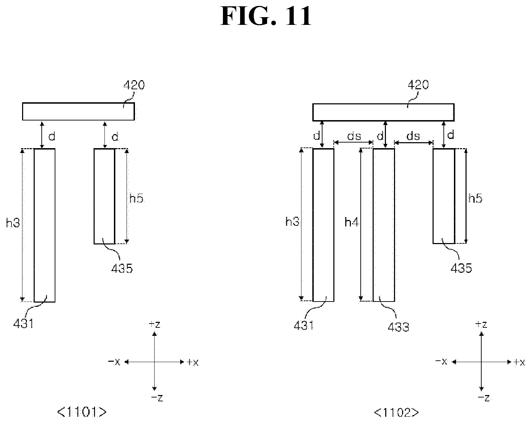

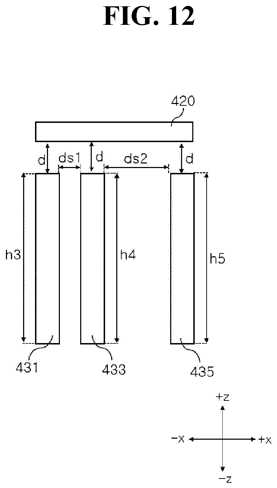

Referring to FIGS. 11 and 12, the lower electrode assembly 430 may include the first lower electrode 431 and the second lower electrode 435. The first lower electrode 431 and the second lower electrode 435 may be formed to extend vertically.

The first lower electrode 431 and the second lower electrode 435 may be disposed to be spaced apart from the shield electrode 420 in the vertical direction by a certain distance d. The first lower electrode 431 and the second lower electrode 435 may be disposed side by side along the horizontal direction.

Referring to reference numeral 1101 of FIG. 11, the third height h3 of the first lower electrode 431 may be different from the fifth height h5 of the second lower electrode 435.

The capacitance corresponding to the first lower electrode 431 and the capacitance corresponding to each second lower electrode 435 may be detected by using the self-capacitance method. At this time, since the third height h3 of the first lower electrode 431 and the fifth height h5 of the second lower electrode 435 are different, the capacitance corresponding to the first lower electrode 431 and the capacitance corresponding to the second lower electrode 435 may be different.

Referring to reference numeral 1102 of FIG. 11, the third height h3 of the first lower electrode 431 may be different from the fifth height h5 of the second lower electrode 435.

A ground electrode 433 may be disposed between the first lower electrode 431 and the second lower electrode 435.

The capacitance corresponding to the first lower electrode 431 and the capacitance corresponding to each second lower electrode 435 may be detected by using the mutual capacitance method for the ground electrode 433. At this time, since the third height h3 of the first lower electrode 431 and the fifth height h5 of the second lower electrode 435 are different, the capacitance corresponding to the first lower electrode 431 and the capacitance corresponding to the second lower electrode 435 may be different.

Referring to FIG. 12, the third height h3 of the first lower electrode 431 may correspond to the fifth height h5 of the second lower electrode 435.

The ground electrode 433 may be disposed between the first lower electrode 431 and the second lower electrode 435. In the horizontal direction, the distance ds1 between the first lower electrode 431 and the ground electrode 433 and the distance ds2 between the second lower electrode 435 and the ground electrode 433 may be different.

The capacitance corresponding to the first lower electrode 431 and the capacitance corresponding to each second lower electrode 435 may be detected by using the mutual capacitance method for the ground electrode 433. At this time, since the distance dsl between the first lower electrode 431 and the ground electrode 433 and the distance ds2 between the second lower electrode 435 and the ground electrode 433 are different, the capacitance corresponding to the first lower electrode 431 and the capacitance corresponding to the second lower electrode 435 may be different.

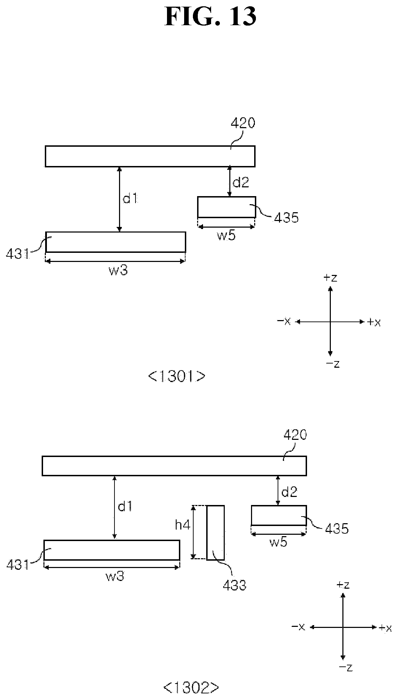

Referring to FIG. 13, the lower electrode assembly 430 may include the first lower electrode 431 and the second lower electrode 435. The first lower electrode 431 and the second lower electrode 435 may be formed to extend horizontally.

The third width w3 of the first lower electrode 431 and the fifth width w5 of the second lower electrode 435 may correspond to each other, or may be different.

The first lower electrode 431 may be disposed to be spaced apart from the shield electrode 420 in the vertical direction by a first distance d1. The second lower electrode 435 may be disposed to be spaced apart from the shield electrode 420 in the vertical direction by a second distance d2. That is, the depth of the fluid directed downward, corresponding to the positions where the first lower electrode 431 and the second lower electrode 435 are respectively disposed, may be different.

Referring to reference numeral 1301, the capacitance corresponding to the first lower electrode 431 and the capacitance corresponding to the second lower electrode 435 may be detected by using a self-capacitance method. At this time, since the depth of the fluid corresponding to the positions where the first lower electrode 431 and the second lower electrode 435 are respectively disposed are different, the capacitance corresponding to the first lower electrode 431 and the capacitance corresponding to the second lower electrode 435 may be different.

Referring to reference numeral 1302, a ground electrode 433 may be disposed between the first lower electrode 431 and the second lower electrode 435 in the horizontal direction.

When the first distance d1 between the first lower electrode 431 and the shield electrode 420 exceeds the second distance d2 between the second lower electrode 435 and the shield electrode 420, the fourth height h4 of the ground electrode 433 may be greater than or equal to the distance between the lower surface of the first lower electrode 431 and the upper surface of the second lower electrode 435.

The capacitance corresponding to the first lower electrode 431 and the capacitance corresponding to each second lower electrode 435 may be detected by using a mutual capacitance method for the ground electrode 433. At this time, since the depth of the fluid corresponding to the positions where the first lower electrode 431 and the second lower electrode 435 are respectively disposed is different, the capacitance corresponding to the first lower electrode 431 and the capacitance corresponding to the second lower electrode 435 may be different.

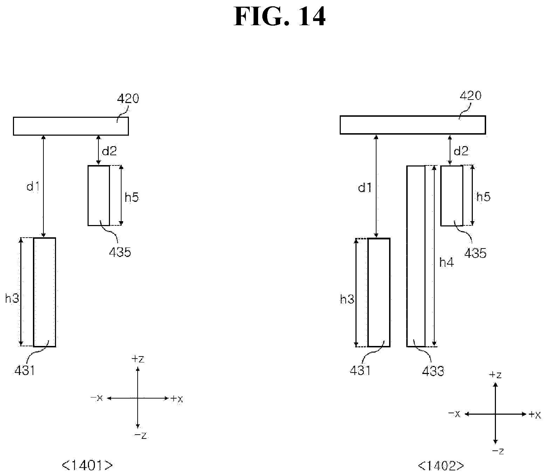

Referring to FIG. 14, the lower electrode assembly 430 may include the first lower electrode 431 and the second lower electrode 435. The first lower electrode 431 and the second lower electrode 435 may be formed to extend vertically.

The third height h3 of the first lower electrode 431 and the fifth height h5 of the second lower electrode 435 may correspond to each other, or may be different.

The first lower electrode 431 may be disposed to be spaced apart from the shield electrode 420 in the vertical direction by a first distance d1. The second lower electrode 435 may be disposed to be spaced apart from the shield electrode 420 in the vertical direction by a second distance d2. That is, the depth of the fluid directed downward, corresponding to the positions where the first lower electrode 431 and the second lower electrode 435 are respectively disposed, may be different.

Referring to reference numeral 1401, the capacitance corresponding to the first lower electrode 431 and the capacitance corresponding to the second lower electrode 435 may be detected by using a self-capacitance method. At this time, since the depth of the fluid corresponding to the positions where the first lower electrode 431 and the second lower electrode 435 are respectively disposed are different, the capacitance corresponding to the first lower electrode 431 and the capacitance corresponding to the second lower electrode 435 may be different.

Referring to reference numeral 1402, a ground electrode 433 may be disposed between the first lower electrode 431 and the second lower electrode 435 in the horizontal direction.

When the first distance d1 between the first lower electrode 431 and the shield electrode 420 exceeds the second distance d2 between the second lower electrode 435 and the shield electrode 420, the fourth height h4 of the ground electrode 433 may be greater than or equal to the distance between the lower surface of the first lower electrode 431 and the upper surface of the second lower electrode 435.

The capacitance corresponding to the first lower electrode 431 and the capacitance corresponding to each second lower electrode 435 may be detected by using a mutual capacitance method for the ground electrode 433. At this time, since the depth of the fluid corresponding to the positions where the first lower electrode 431 and the second lower electrode 435 are respectively disposed is different, the capacitance corresponding to the first lower electrode 431 and the capacitance corresponding to the second lower electrode 435 may be different.

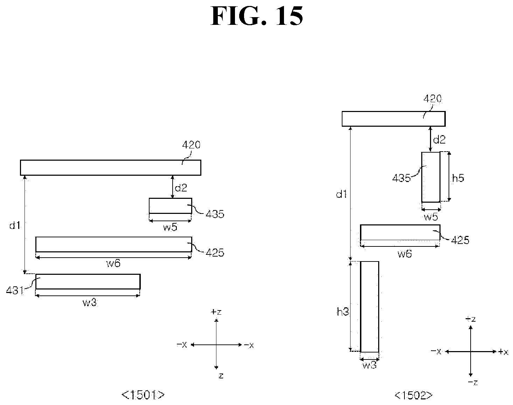

Referring to FIG. 15, the lower electrode assembly 430 may include the first lower electrode 431 and the second lower electrode 435. The first lower electrode 431 and the second lower electrode 435 may be formed to extend horizontally.

The third width w3 of the first lower electrode 431 and the fifth width w5 of the second lower electrode 435 may correspond to each other, or may be different. The third height h3 of the first lower electrode 431 and the fifth height h5 of the second lower electrode 435 may correspond to each other, or may be different.

The first lower electrode 431 may be disposed vertically apart from the shield electrode 420 by a first distance d1. The second lower electrode 435 may be disposed vertically apart from the shield electrode 420 by a second distance d2. That is, the depths of the fluids directed downward, corresponding to the positions where the first lower electrode 431 and the second lower electrode 435 are disposed, may be different.

The electrode assembly 310 may further include a sub-shield electrode 425. The sub-shield electrode 425 may be formed to extend horizontally.

The sub-shield electrode 425 may be formed to have a sixth width w6 along the left-right direction. When the first lower electrode 431 is disposed on the left side and the second lower electrode 435 is disposed on the right side, the sixth width w6 of the sub-shield electrode 425 may be greater than or equal to the distance between the left surface of the first lower electrode 431 and the right surface of the second lower electrode 435.

The sub-shield electrode 425 may be formed to have a sixth length along the front-rear direction. The sixth length of the sub-shield electrode 425 may correspond to the third length l3 of the first lower electrode 431 and the fifth length l5 of the second lower electrode 435.

According to an embodiment, a sensing voltage may be applied to the sub-shield electrode 425. That is, the sub-shield electrode 425 may be configured for active shielding.

The capacitance corresponding to the first lower electrode 431 and the capacitance corresponding to each second lower electrode 435 may be detected by using a self-capacitance method. At this time, since the depth of the fluid corresponding to the positions where the first lower electrode 431 and the second lower electrode 435 are disposed respectively is different, the capacitance corresponding to the first lower electrode 431 and the capacitance corresponding to the second lower electrode 435 may be different.

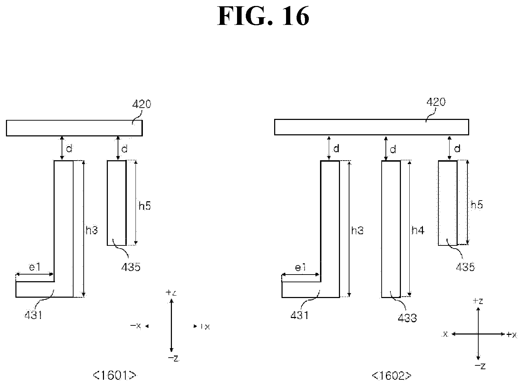

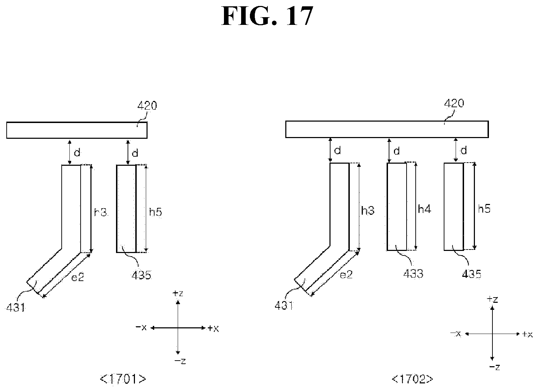

Referring to FIGS. 16 and 17, the lower electrode assembly 430 may include the first lower electrode 431 and the second lower electrode 435. The first lower electrode 431 and the second lower electrode 435 may be formed to extend vertically.

The third height h3 of the first lower electrode 431 and the fifth height h5 of the second lower electrode 435 may correspond to each other, or may be different.

The first lower electrode 431 and the second lower electrode 435 may be disposed vertically apart from the shield electrode 420 by a certain distance d. The first lower electrode 431 and the second lower electrode 435 may be disposed side by side along the horizontal direction.

The shape of the first lower electrode 431 and the shape of the second lower electrode 435 may be different. The shape of the first lower electrode 431 and the shape of each second lower electrode 435 may correspond to the surrounding environment in which the sensor 300 is disposed, the internal structure of the sensor 300 in which the lower electrode assembly 430 is disposed, etc.

For example, referring to FIG. 16, the lower one end of the first lower electrode 431 may be formed to extend in the left direction by a first extension length el. For example, referring to FIG. 17, the first lower electrode 431 may be formed to extend in the left direction and the lower direction by a second extension length e2.

Referring to the reference numeral 1601 of FIG. 16 and the reference numeral 1701 of FIG. 17, the capacitance corresponding to the first lower electrode 431 and the capacitance corresponding to each second lower electrode 435 may be detected by using a self-capacitance method. At this time, since the shape of the first lower electrode 431 and the shape of the second lower electrode 435 are different, the capacitance corresponding to the first lower electrode 431 and the capacitance corresponding to the second lower electrode 435 may be different.

Referring to the reference numeral 1602 of FIG. 16 and the reference numeral 1702 of FIG. 17, a ground electrode 433 may be disposed between the first lower electrode 431 and the second lower electrode 435.

The capacitance corresponding to the first lower electrode 431 and the capacitance corresponding to each second lower electrode 435 may be detected by using a mutual capacitance method for the ground electrode 433. At this time, since the shape of the first lower electrode 431 and the shape of the second lower electrode 435 are different, the capacitance corresponding to the first lower electrode 431 and the capacitance corresponding to the second lower electrode 435 may be different.

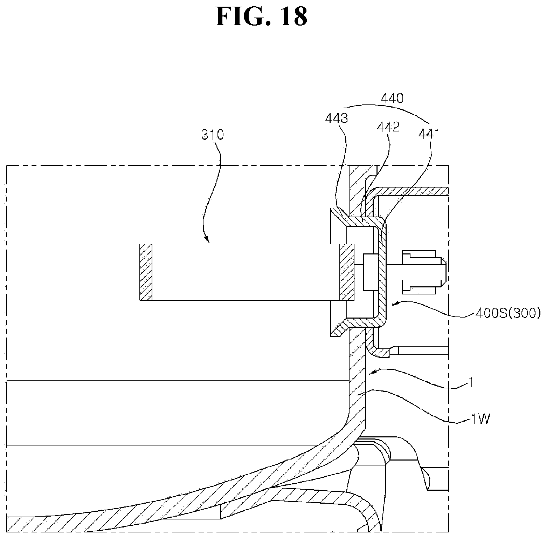

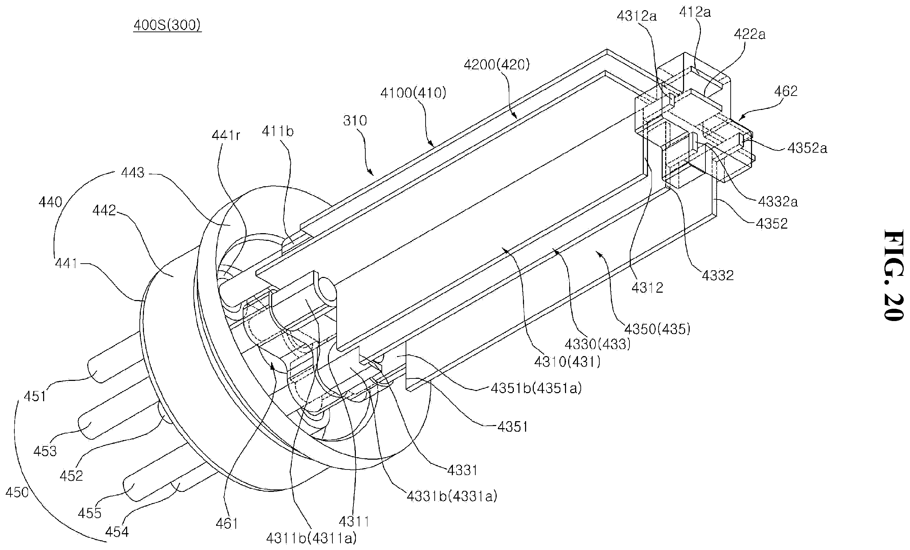

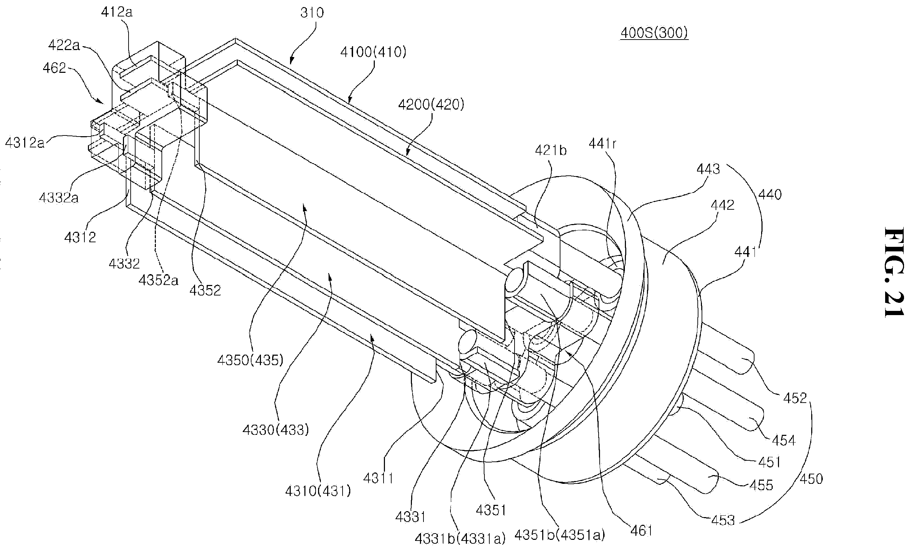

FIGS. 18 to 23 are diagrams illustrating a sensing module of a sensor according to an embodiment of the present disclosure.

Referring to FIGS. 18 to 21, the sensor 300 may include a sensing module 400S. The sensing module 400S may include the electrode assembly 310, a housing 440, and a pin assembly 450.

For example, the electrode assembly 310 may be installed in a lower portion of the compressor 1 (see FIGS. 1A and 1B). The electrode assembly 310 may be inserted into the compressor 1, and the pin assembly 450 may be electrically connected to the processing circuit 320 (see FIG. 3) outside the compressor 1 via a cable.

The housing 440 may have an overall cap shape. The housing 440 may be referred to as a cap 440. The housing 440 may be opened toward the electrode assembly 310. A bottom 441 of the housing 440 may have a disc shape. Alternatively, the bottom 441 of the housing 440 may have an angular shape (for example, a square or a pentagon). A side wall 442 of the housing 440 may extend along an edge of the bottom 441 of the housing 440. A flange 443 of the housing 440 may protrude from a side surface of the side wall 442 and may extend along the side wall 442. The diameter (width) of the flange 443 of the housing 440 may be larger than the diameter (width) of the side wall 442 of the housing 440. The flange 443 of the housing 440 may be adjacent to a distal end of the side wall 442. The housing 440 may penetrate a body 1W of the compressor 1. The flange 443 of the housing 440 may face an inner side surface of the body 1W of the compressor 1, and the side wall 442 of the housing 440 may be welded to the body 1W of the compressor 1.

The electrode assembly 310 may face the bottom 441 of the housing 440. The electrode assembly 310 may be spaced apart from the bottom 441 of the housing 440 and may extend in a direction intersecting the bottom 441. The electrode assembly 310 may include a material having electrical conductivity, such as metal. The electrode assembly 310 may include the upper electrode 410, the shield electrode 420, and the lower electrode assembly 430. The upper electrode 410 may be referred to as a liquid level detection unit 410. The lower electrode assembly 430 may be referred to as a concentration detection unit 430.

The upper electrode 410 may have a plate shape extending in a horizontal direction. The upper electrode 410 may have an overall rectangular shape. The upper electrode 410 may have two long sides that are opposite to each other and two short sides that are opposite to each other. A first protruding portion 411a may protrude from a first short side 411 of the upper electrode 410 and form one end of the upper electrode 410. A second protruding portion 412a may protrude from a second short side 412 of the upper electrode 410 and form the other end of the upper electrode 410. A portion of the upper electrode 410 between the protrusions 411a, 412a may be referred to as an upper body 4100 and may have a length and width greater than the protrusions 411a, 412a.

The shield electrode 420 may be spaced downward from the upper electrode 410. The shield electrode 420 may have a plate shape extending in a horizontal direction. The shield electrode 420 may have two long sides that are opposite to each other and two short sides that are opposite to each other. A first protruding portion 421a may protrude from a first short side 421 of the shield electrode 420 and form one end of the shield electrode 420. A second protruding portion 422a may protrude from a second short side 422 of the shield electrode 420 and form the other end of the shield electrode 420. The portion of the shield electrode 420 between the protrusions 421a, 422a may be referred to as a shield body 4200 and may have a length and width greater than the protrusions 421a, 422a. The length and width of the shield body 4200 may be greater than or equal to the length and width of the upper body 4100. In other words, the area of the shield body 4200 may be greater than or equal to the area of the upper body 4100. The shield body 4200 may cover the lower surface of the upper body 4100.

The lower electrode assembly 430 may be spaced downward from the shield electrode 420. The lower electrode assembly 430 may have a plate shape extending in a direction intersecting the shield electrode 420. The lower electrode assembly 430 may be extended long in a lengthwise direction of the shield electrode 420. Herein, the lengthwise direction of the shield electrode 420 may be a direction parallel to long sides of the shield electrode 420. The electrodes of the lower electrode assembly 430 may be spaced apart from each other in a horizontal direction. The lower electrode assembly 430 may include the first lower electrode 431, the second lower electrode 435, and the ground electrode 433.

The first lower electrode 431 may have a plate shape perpendicular to the shield electrode 420. The first lower electrode 431 may have an overall rectangular shape. The first lower electrode 431 may have two long sides that are opposite to each other and two short sides that are opposite to each other. The first long side of the first lower electrode 431 may be adjacent to the lower surface of the shield electrode 420. A first protruding portion 4311a may protrude from a first short side 4311 of the first lower electrode 431 and form one end of the first lower electrode 431. A second protruding portion 4312a may protrude from a second short side 4312 of the first lower electrode 431 and form the other end of the first lower electrode 431. A portion of the first lower electrode 431 between the protruding portions 4311a, 4312a may be referred to as a first lower body 4310 and may have a length and width greater than the protruding portions 4311a, 4312a. The length of the first lower body 4310 may be less than or equal to the length of the shield body 4200, and the shield body 4200 may cover an upper edge of the first lower body 4310.

The second lower electrode 435 may have a plate shape perpendicular to the shield electrode 420. The second lower electrode 435 may be spaced apart from the first lower electrode 431 in a horizontal direction. The second lower electrode 435 may have an overall rectangular shape. The second lower electrode 435 may have two long sides that are opposite to each other and two short sides that are opposite to each other. The first long side of the second lower electrode 435 may be adjacent to the lower surface of the shield electrode 420. A first protruding portion 4351a may protrude from a first short side 4351 of the second lower electrode 435 and form one end of the second lower electrode 435. A second protruding portion 4352a may protrude from a second short side 4352 of the second lower electrode 435 and form the other end of the second lower electrode 435. A portion of the second lower electrode 435 between the protruding portions 4351a, 4352a may be referred to as a second lower body 4350, and may have a length and width greater than the protruding portions 4351a, 4352a. The length of the second lower body 4350 may be less than or equal to the length of the shield body 4200, and the shield body 4200 may cover an upper edge of the second lower body 4350.

The ground electrode 433 may have a plate shape perpendicular to the shield electrode 420. The ground electrode 433 may be disposed between the first lower electrode 431 and the second lower electrode 435, and may be horizontally spaced from the first lower electrode 431 and the second lower electrode 435. The ground electrode 433 may have an overall rectangular shape. The ground electrode 433 may have two long sides that are opposite to each other and two short sides that are opposite to each other. The first long side of the ground electrode 433 may be adjacent to the lower surface of the shield electrode 420. A first protruding portion 4331a may protrude from a first short side 4331 of the ground electrode 433 and form one end of the ground electrode 433. A second protruding portion 4332a may protrude from a second short side 4332 of the ground electrode 433 and form the other end of the ground electrode 433. A portion of the ground electrode 433 between the protruding portions 4331a, 4332a may be referred to as a ground body 4330 and may have a greater length and width than the protruding portions 4331a, 4332a. The length of the ground body 4330 may be less than or equal to the length of the shield body 4200, and the shield body 4200 may cover an upper edge of the ground body 4330.

Accordingly, the shield electrode 420 may be disposed between the upper electrode 410 and the lower electrode assembly 430, and may shield the upper electrode 410 from the lower electrode assembly 430, while shielding the lower electrode assembly 430 from the upper electrode 410.

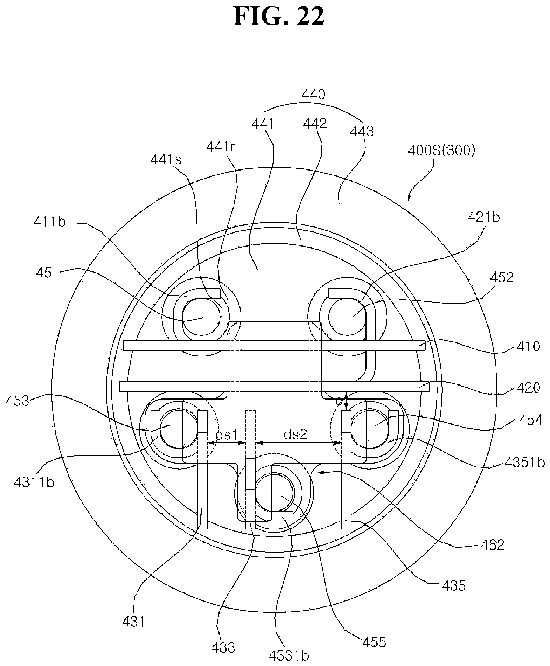

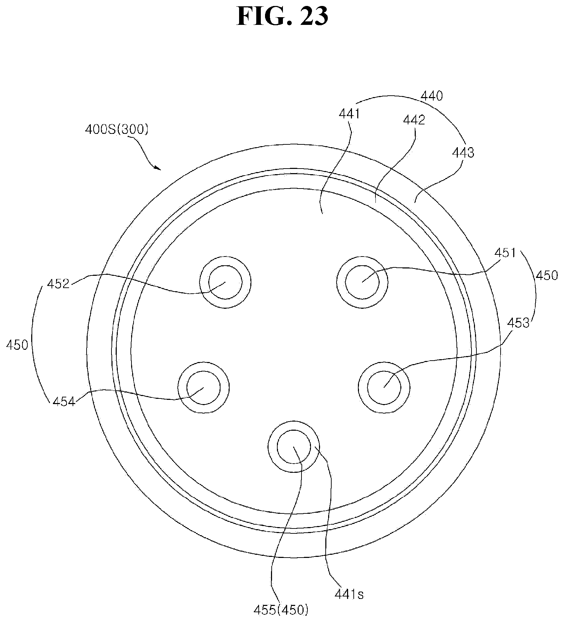

Referring to FIGS. 22 and 23, the pin assembly 450 may penetrate the bottom 441 of the housing 440. The pin assembly 450 may include a material having electrical conductivity, such as metal. The side wall 442 of the housing 440 may surround the pin assembly 450. A rib 441r protruding from an inner side surface of the bottom 441 may surround a portion of an outer side surface of the pin assembly 450. A sealant 441s may block a gap between the outer side surface of the pin assembly 450 and the inner side surface of the rib 441r. The sealant 441s may be made of an electrically insulating material such as glass. The sealant 441s may be referred to as an insulating material 441s or a glass frit 441s.

The pin assembly 450 may include a plurality of pins. The pins may be electrically conductive. The pins may be referred to as conductive pins. The number of the pins may be equal to the number of electrodes of the electrode assembly 310. Alternatively, the number of the pins may be one less than the number of electrodes of the electrode assembly 310. In other words, the pin assembly 450 may have pins connected to the upper electrode 410 and the lower electrode assembly 430, and may or may not have pins connected to the shield electrode 420.

For example, the pin assembly 450 may include a first pin 451, a second pin 452, a third pin 453, a fourth pin 454, and a fifth pin 455. The first to fifth pins 451, 452, 453, 454, 455 may extend in a direction intersecting the bottom 441 of the housing 440. The first to fifth pins 451, 452, 453, 454, 455 may be disposed adjacent to an edge of the bottom 441 of the housing 440 and along the edge.

In other words, the first to fifth pins 451, 452, 453, 454, 455 may be disposed radially with respect to the center of the bottom 441 of the housing 440. The first pin 451 may be electrically connected to the upper electrode 410. The second pin 452 may be electrically connected to the shield electrode 420. The third pin 453 may be electrically connected to the first lower electrode 431. The fourth pin 454 may be electrically connected to the second lower electrode 435. The fifth pin 455 may be electrically connected to the ground electrode 433.

The sensing voltage may be applied to each of the upper electrode 410, the shield electrode 420, the first lower electrode 431 and the second lower electrode 435 through the first pin 451, the second pin 452, the third pin 453 and the fourth pin 454, respectively. The ground electrode may be applied to the ground electrode 433 through the fifth pin 455. In this case, the shield electrode 420 may provide active shielding.

Alternatively, the second pin 452 may be omitted so that no voltage is applied to the shield electrode 420. In this case, the shield electrode 420 may provide passive shielding.

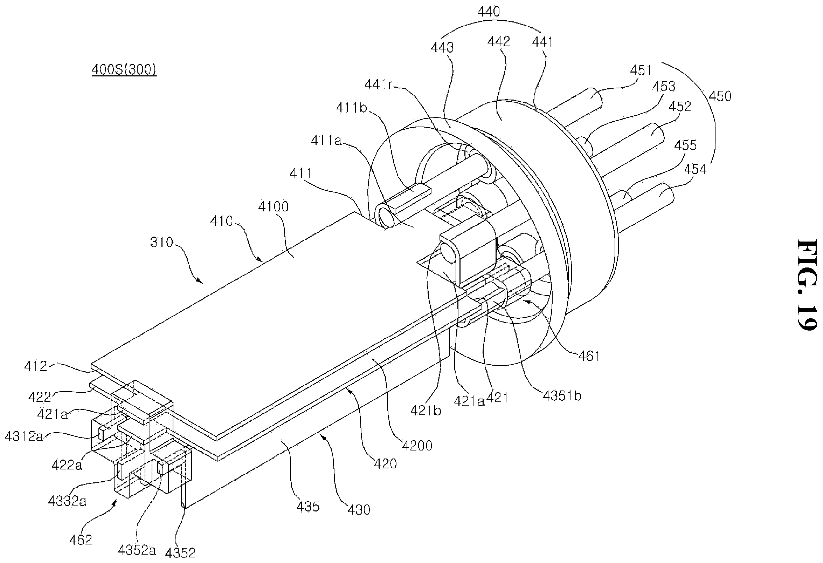

Referring again to FIGS. 19 to 21, the first and second pins 451, 452 may be disposed above the upper electrode 410, and the third to fifth pins 453, 454, 455 may be disposed below the shield electrode 420.

A portion of the first pin 451 may extend from the bottom 441 of the housing 440 toward an upper space of the upper electrode 410, and the remainder of the first pin 451 may extend in an opposite direction of a portion thereof. The first pin 451 may be adjacent to the first protruding portion 411a of the upper electrode 410. A first coupling portion 411b may protrude from the first protruding portion 411a to contact and be coupled to the first pin 451. The first coupling portion 411b may be bent from the first protruding portion 411a to surround a portion of the first pin 451 and may be welded to the first pin 451. In other words, the upper electrode 410 may be hung on the first pin 451.

A portion of the second pin 452 may extend from the bottom 441 of the housing 440 toward an upper space of the upper electrode 410, and the remainder of the second pin 452 may extend in an opposite direction of the portion thereof. The second pin 452 may be adjacent to the first protruding portion 421a of the shield electrode 420. A first coupling portion 421b may be protruded from the first protruding portion 421a to contact and be coupled to the second pin 452. The first coupling portion 421b may be bent from the first protruding portion 421a to surround a portion of the second pin 452 and may be welded to the second pin 452. In other words, the shield electrode 420 may be hung on the second pin 452.

A portion of the third pin 453 may extend from the bottom 441 of the housing 440 toward a lower space of the shield electrode 420, and the remainder of the third pin 453 may extend in an opposite direction of the portion thereof. The third pin 453 may be adjacent to the first lower electrode 431. A first coupling portion 4311b may be protruded from the first protruding portion 4311a to contact and be coupled to the third pin 453. The first coupling portion 4311b may be bent from the first protruding portion 4311a to surround a portion of the third pin 453 and may be welded to the third pin 453. In other words, the first lower electrode 431 may be hung on the third pin 453.

A portion of the fourth pin 454 may extend from the bottom 441 of the housing 440 toward a lower space of the shield electrode 420, and the remainder of the fourth pin 454 may extend in an opposite direction of the portion thereof. The fourth pin 454 may be adjacent to the second lower electrode 435. A first coupling portion 4351b may be protruded from the first protruding portion 4351a to contact and be coupled to the fourth pin 454. The first coupling portion 4351b may be bent from the first protruding portion 4351a to surround a portion of the fourth pin 454 and may be welded to the fourth pin 454. In other words, the second lower electrode 435 may be hung on the fourth pin 454.

A portion of the fifth pin 455 may extend from the bottom 441 of the housing 440 toward a lower space of the shield electrode 420, and the remainder of the fifth pin 455 may extend in an opposite direction of the portion thereof. The fifth pin 455 may be adjacent to the ground electrode 433. A first coupling portion 4331b may be protruded from the first protruding portion 4331a to contact and be coupled to the fifth pin 455. The first coupling portion 4331b may be bent from the first protruding portion 4331a to surround a portion of the fifth pin 455 and may be welded to the fifth pin 455. In other words, the ground electrode 433 may be hung on the fifth pin 455.

A first holder 461 may be disposed between the bottom 441 of the housing 440 and the electrode assembly 310. The first holder 461 may include an electrically insulating material such as plastic. For example, the first holder 461 may be polyphenylene sulfide (PPS). At least a portion of the plurality of pins 451, 452, 453, 454, 455 may penetrate the first holder 461. For example, the third to fifth pins 453, 454, 455 may penetrate the first holder 461. Accordingly, the first holder 461 may minimize the movement of the pin assembly 450. The first holder 461 may be referred to as a pin fixing portion 461 or a first electrode fixing portion 461.

A second holder 462 may be located opposite the first holder 461 with respect to the electrode assembly 310. The second holder 462 may include an electrically insulating material such as plastic. For example, the second holder 462 may be polyphenylene sulfide (PPS). At least some of the plurality of electrodes 410, 420, 431, 433, 435 may be inserted into the second holder 462. All of the plurality of electrodes 410, 420, 431, 433, 435 may be inserted into the second holder 461. Accordingly, the second holder 462 may minimize the movement of the electrode assembly 310. The second holder 462 may be referred to as a second electrode fixing portion 462.

Referring again to FIG. 22, the pin assembly 450 may penetrate the bottom 441 of the housing 440, and the electrode assembly 310 may be coupled to the pin assembly 450. The electrode assembly 310 may be disposed inside the edge of the housing 440. The electrode assembly 310 may be disposed inside the circle formed by the side wall 442 of the housing 440. Accordingly, during the process of welding the housing 440 to the compressor 1 (see FIG. 18), the short circuit of the sensor module 400S may be prevented. In addition, the increase in the size of the sensor module 400S may be minimized by limiting the width (diameter) of the sensor module 400S to the width (diameter) of the housing 440.

The first lower electrode 431, the second lower electrode 435, and the ground electrode 433 of the lower electrode assembly 430 of FIG. 22 may be spaced apart from the shield electrode 420 by a predetermined distance d downward. In a horizontal direction, a distance dsl between the first lower electrode 431 and the ground electrode 433 may be different from a distance ds2 between the second lower electrode 435 and the ground electrode 433. The distance ds1 may be smaller than the distance ds2. The width of the first lower electrode 431 (see a height h3 of FIG. 12), the width of the second lower electrode 435 (see a height h5 of FIG. 12), and the width of the ground electrode 433 (see a height h4 of FIG. 12) may correspond to one another. Herein, in a vertical direction, the length of the lower electrode assembly 430 may be referred to as the width or height (see h3, h4, h5 of FIG. 12). The shape and disposition of the lower electrode assembly 430 of FIG. 22 may correspond to the shape and disposition of the lower electrode assembly 431, 433, 435 described above with reference to FIG. 12.

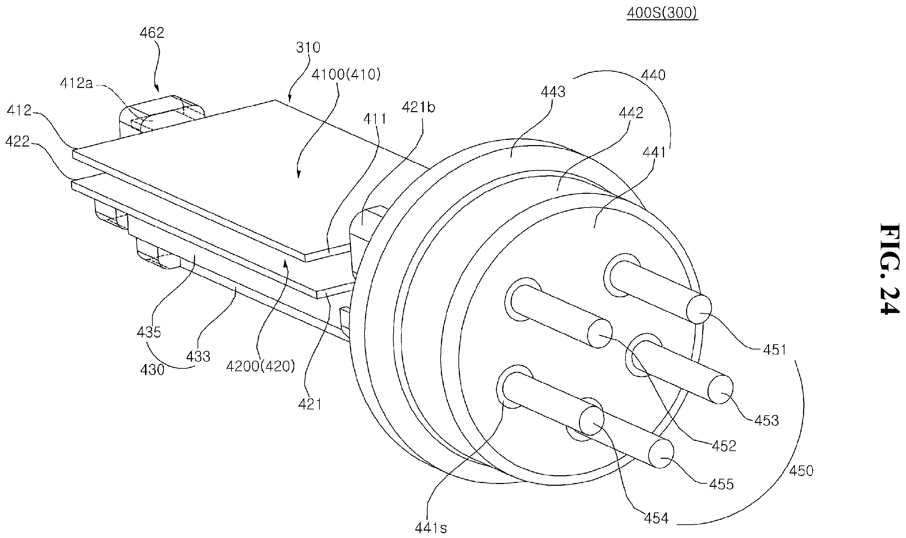

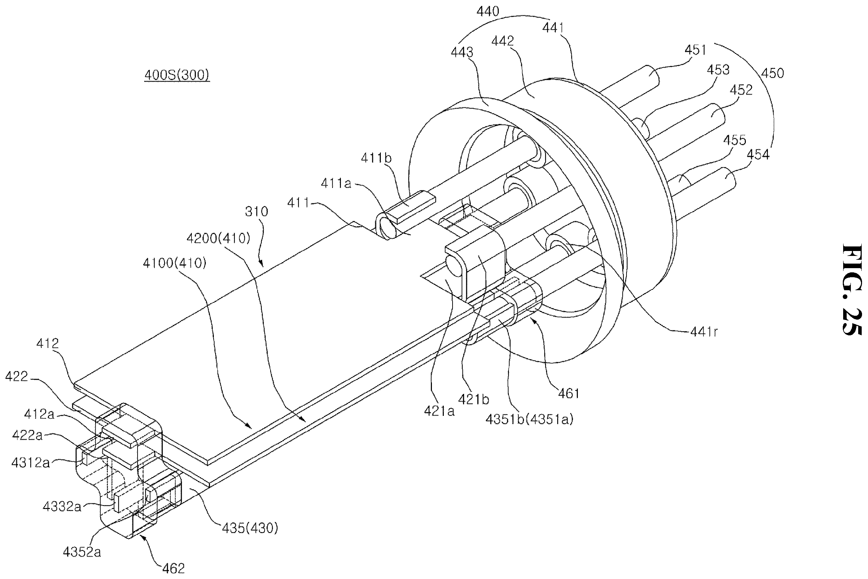

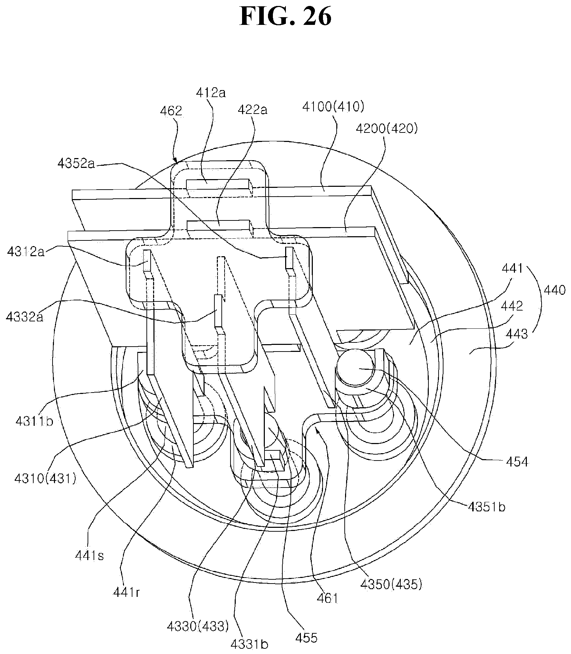

FIGS. 24 to 27 are diagrams illustrating a sensing module of a sensor according to an embodiment of the present disclosure.

Referring to FIGS. 24 to 27, the sensor 300 may include the sensing module 400S. The shape and disposition of the lower electrode assembly 430 of the sensing module 400S of FIGS. 24 to 27 may be different from the shape and disposition of the lower electrode assembly 430 of the sensing module 400S of FIGS. 18 to 23. The housing 440, the pin assembly 450, the upper electrode 410, and the shield electrode 420 of the sensing module 400S of FIGS. 24 to 27 may be the same as the housing 440, the pin assembly 450, the upper electrode 410, and the shield electrode 420 of the sensing module 400S of FIGS. 18 to 23.

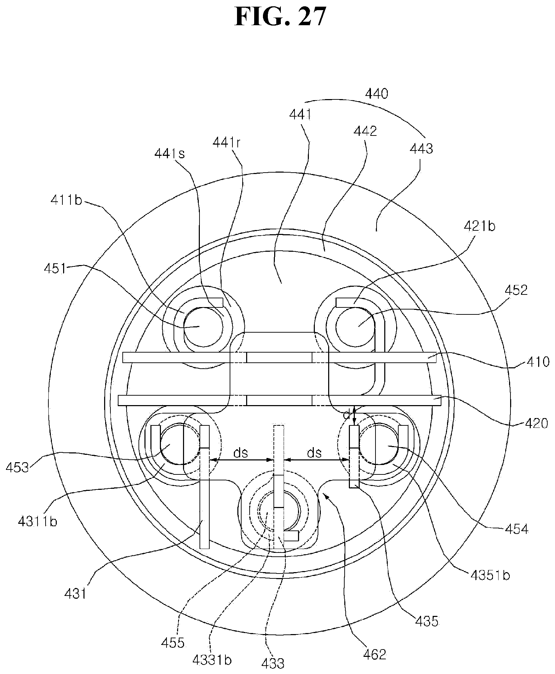

The first lower electrode 431, the second lower electrode 435, and the ground electrode 433 of the lower electrode assembly 430 of FIG. 27 may be spaced apart from the shield electrode 420 by a predetermined distance d downward. In a horizontal direction, a distance ds between the first lower electrode 431 and the ground electrode 433 may correspond to a distance ds between the second lower electrode 435 and the ground electrode 433. The width of the first lower electrode 431 (see the height h3 of reference numeral 1102 of FIG. 11) and the width of the ground electrode 433 (see the height h4 of reference numeral 1102 of FIG. 11) may correspond to each other. The width of the second lower electrode 435 (see the height h5 of reference numeral 1102 of FIG. 11) may be smaller than the width of the ground electrode 433 (see the height h4 of reference numeral 1102 of FIG. 11). Herein, in a vertical direction, the length of the lower electrode assembly 430 may be referred to as the width or the height (see h3, h4, h5 of reference numeral 1102 of FIG. 11). The shape and disposition of the lower electrode assembly 430 of FIG. 27 may correspond to the shape and disposition of the lower electrode assembly 431, 433, 435 described above with reference to reference numeral 1102 of FIG. 6A or FIG. 11.

The lower electrode assembly 430 of the sensing module 400S of FIGS. 18 to 27 may be changed to the lower electrode assembly 431, 433, 435 of reference numeral 1402 of FIG. 14, the lower electrode assembly 431, 433, 435 of reference numeral 1602 of FIG. 16, or the lower electrode assembly 431, 433, 435 of reference numeral 1702 of FIG. 17. In this case, the number of pins of the pin assembly 450 may be five, and may be electrically connected to the upper electrode 410, the shield electrode 420, and the lower electrode assembly 431, 433, 435. Alternatively, the pin connected to the shield electrode 420 may be omitted, in which case the number of pins of the pin assembly 450 may be four.

The lower electrode assembly 430 of the sensing module 400S of FIGS. 18 to 27 may be changed to the lower electrode assembly 431, 433 of FIG. 5A. In this case, the number of pins of the pin assembly 450 may be four, and may be electrically connected to the upper electrode 410, the shield electrode 420, and the lower electrode assembly 431, 433. Alternatively, the pin connected to the shield electrode 420 may be omitted, in which case the number of pins of the pin assembly 450 may be three.

The lower electrode assembly 430 of the sensing module 400S of FIGS. 18 to 27 may be changed to the lower electrode assembly 431, 435 of reference numeral 1101 of FIG. 11, the lower electrode assembly 431, 435 of reference numeral 1401 of FIG. 14, the lower electrode assembly 431, 435 of reference numeral 1601 of FIG. 16, or the lower electrode assembly 431, 435 of reference numeral 1701 of FIG. 17. In this case, the number of pins of the pin assembly 450 may be four, and may be electrically connected to the upper electrode 410, the shield electrode 420, and the lower electrode assembly 431, 435. Alternatively, the pin connected to the shield electrode 420 may be omitted, in which case the number of pins of the pin assembly 450 may be three.

The lower electrode assembly 430 of the sensing module 400S of FIGS. 18 to 27 may be changed to the lower electrode assembly 431 of FIG. 4A. In this case, the number of pins of the pin assembly 450 may be three, and may be electrically connected to the upper electrode 410, the shield electrode 420, and the lower electrode assembly 431. Alternatively, the pin connected to the shield electrode 420 may be omitted, in which case the number of pins of the pin assembly 450 may be two.

The lower electrode assembly 430 of the sensing module 400S of FIGS. 18 to 27 may be changed to the lower electrode assembly 431, 435 and the sub-shield electrode 425 of reference numeral 1502 of FIG. 15. In this case, the number of pins of the pin assembly 450 may be five, and may be electrically connected to the upper electrode 410, the shield electrode 420, the lower electrode assembly 431, 435, and the sub-shield electrode 425. Alternatively, the pins connected to the shield electrode 420 or the sub-shield electrode 425 may be omitted, in which case the number of pins of the pin assembly 450 may be four. Alternatively, the pins connected to the shield electrode 420 and the sub-shield electrode 425 may be omitted, in which case the number of pins of the pin assembly 450 may be three.

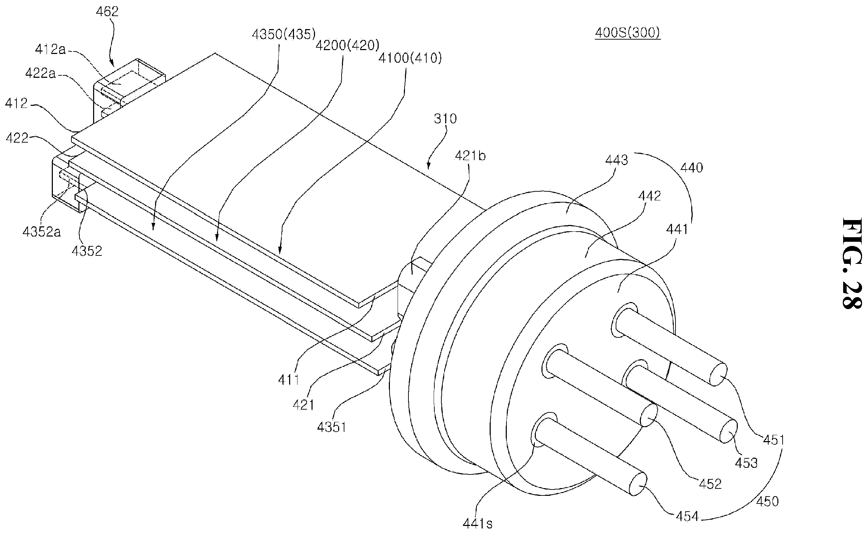

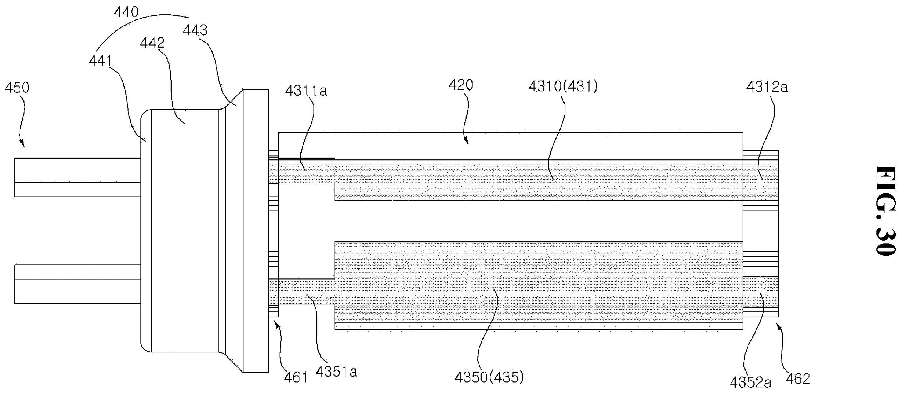

FIGS. 28 to 31 are diagrams illustrating a sensing module of a sensor according to an embodiment of the present disclosure.

Referring to FIGS. 28 to 31, the sensor 300 may include the sensing module 400S. The shape and disposition of the lower electrode assembly 430 of the sensing module 400S of FIGS. 28 to 31 may be different from the shape and disposition of the lower electrode assembly 430 of the sensing module 400S of FIGS. 18 to 27. The housing 440, the pin assembly 450, the upper electrode 410, and the shield electrode 420 of the sensing module 400S of FIGS. 28 to 31 may be the same as the housing 440, the pin assembly 450, the upper electrode 410, and the shield electrode 420 of the sensing module 400S of FIGS. 18 to 27.

Referring to FIGS. 28 to 30, the lower electrode assembly 430 may be spaced downward from the shield electrode 420. The lower electrode assembly 430 may have a plate shape extending in the direction parallel to the shield electrode 420. The lower electrode assembly 430 may be extended long in a lengthwise direction of the shield electrode 420. Here, the lengthwise direction of the shield electrode 420 may be a direction parallel to the long sides of the shield electrode 420. The electrodes of the lower electrode assembly 430 may be spaced apart from each other in a horizontal direction. The lower electrode assembly 430 may include the first lower electrode 431 and the second lower electrode 435.

The first lower electrode 431 may have a plate shape parallel to the shield electrode 420. The first lower electrode 431 may have an overall rectangular shape. The first lower electrode 431 may have two long sides that are opposite to each other and two short sides that are opposite to each other. The upper surface of the first lower electrode 431 may be adjacent to the lower surface of the shield electrode 420. The first protruding portion 4311a may protrude from the first short side 4311 of the first lower electrode 431 and form one end of the first lower electrode 431. The second protruding portion 4312a may protrude from the second short side 4312 of the first lower electrode 431 and form the other end of the first lower electrode 431. A portion of the first lower electrode 431 between the protruding portions 4311a, 4312a may be referred to as the first lower body 4310 and may have a length and width greater than the protruding portions 4311a, 4312a. The length of the first lower body 4310 may be less than or equal to the length of the shield body 4200, and the shield body 4200 may cover an upper surface of the first lower body 4310.

The second lower electrode 435 may have a plate shape parallel to the shield electrode 420. The second lower electrode 435 may have an overall rectangular shape. The second lower electrode 435 may have two long sides that are opposite to each other and two short sides that are opposite to each other. The upper surface of the second lower electrode 435 may be adjacent to the lower surface of the shield electrode 420. The first protruding portion 4351a may protrude from the first short side 4351 of the second lower electrode 435 and form one end of the second lower electrode 435. The second protruding portion 4352a may protrude from the second short side 4352 of the second lower electrode 435 and form the other end of the second lower electrode 435. A portion of the second lower electrode 435 between the protruding portions 4351a, 4352a may be referred to as the second lower body 4350, and may have a length and width greater than the protruding portions 4351a, 4352a. The length of the second lower body 4350 may be less than or equal to the length of the shield body 4200, and the shield body 4200 may cover an upper surface of the second lower body 4350.

Accordingly, the shield electrode 420 may be disposed between the upper electrode 410 and the lower electrode assembly 430, and the upper electrode 410 may shield the lower electrode assembly 430 while shielding the lower electrode assembly 430 from the upper electrode 410.

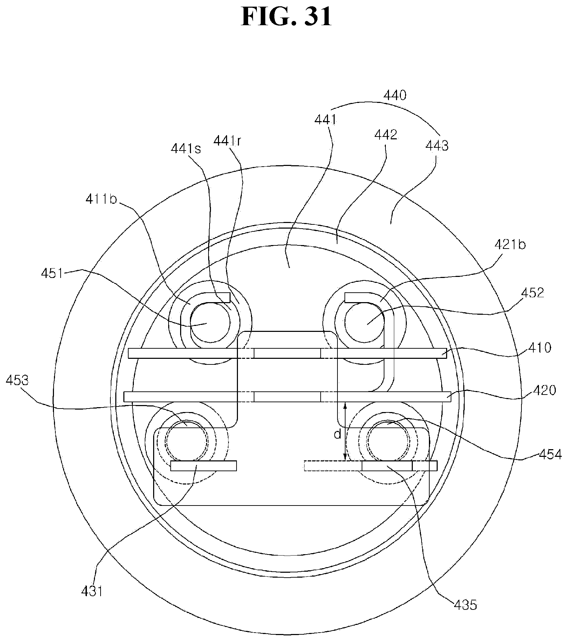

Referring to FIG. 31, the pin assembly 450 may penetrate the bottom 441 of the housing 440. The side wall 442 of the housing 440 may be surrounded by the pin assembly 450. The rib 441r protruding from an inner side surface of the bottom 441 may surround a portion of an outer side surface of the pin assembly 450. The sealant 441s may block a gap between the outer side surface of the pin assembly 450 and the inner side surface of the rib 441r. The sealant 441s may be made of an electrically insulating material such as glass. The sealant 441s may be referred to as the insulating material 441s or the glass frit 441s.

The pin assembly 450 may include the plurality of pins. The pins may be electrically conductive. The number of the pins may be equal to the number of electrodes of the electrode assembly 310. Alternatively, the number of the pins may be one less than the number of electrodes of the electrode assembly 310. In other words, the pin assembly 450 may have pins connected to the upper electrode 410 and the lower electrode assembly 430, and may or may not have pins connected to the shield electrode 420.

For example, the pin assembly 450 may include the first pin 451, the second pin 452, the third pin 453, and the fourth pin 454. The first to fourth pins 451, 452, 453, 454, may extend in a direction intersecting the bottom 441 of the housing 440. The first to fourth pins 451, 452, 453, 454, may be disposed adjacent to an edge of the bottom 441 of the housing 440 and along the edge. In other words, the first to fourth pins 451, 452, 453, 454 may be disposed radially with respect to the center of the bottom 441 of the housing 440. The first pin 451 may be electrically connected to the upper electrode 410. The second pin 452 may be electrically connected to the shield electrode 420. The third pin 453 may be electrically connected to the first lower electrode 431. The fourth pin 454 may be electrically connected to the second lower electrode 435.

The sensing voltage may be applied to each of the upper electrode 410, the shield electrode 420, the first lower electrode 431 and the second lower electrode 435 through the first pin 451, the second pin 452, the third pin 453 and the fourth pin 454, respectively. In this case, the shield electrode 420 may provide active shielding.

Alternatively, the second pin 452 may be omitted so that no voltage is applied to the shield electrode 420. In this case, the shield electrode 420 may provide passive shielding.

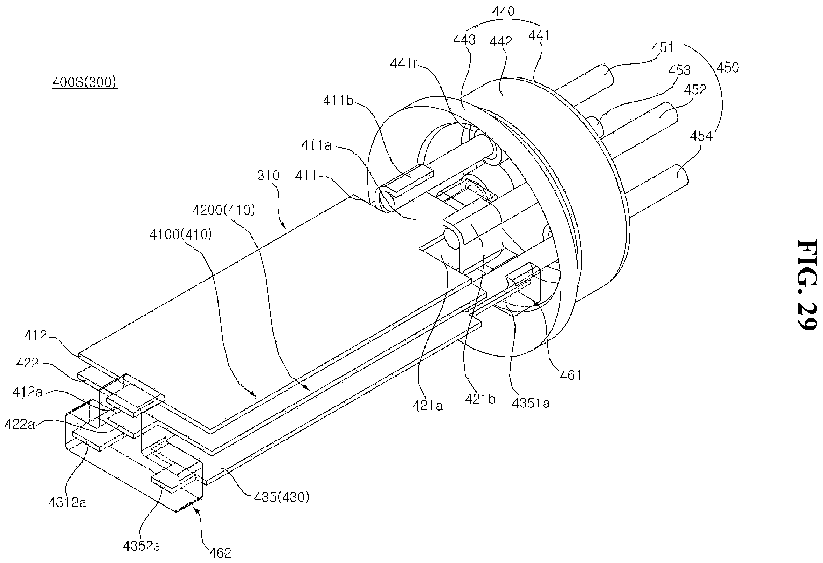

Referring again to FIGS. 29 to 31, the first and second pins 451, 452 may be disposed above the upper electrode 410, and the third to fourth pins 453, 454 may be disposed below the shield electrode 420.

A portion of the first pin 451 may extend from the bottom 441 of the housing 440 toward an upper space of the upper electrode 410, and the remainder of the first pin 451 may extend in an opposite direction of a portion thereof. The first pin 451 may be adjacent to the first protruding portion 411a of the upper electrode 410. The first coupling portion 411b may protrude from the first protruding portion 411a to contact and be coupled to the first pin 451. The first coupling portion 411b may be bent from the first protruding portion 411a to surround a portion of the first pin 451 and may be welded to the first pin 451. In other words, the upper electrode 410 may be hung on the first pin 451.

A portion of the second pin 452 may extend from the bottom 441 of the housing 440 toward an upper space of the upper electrode 410, and the remainder of the second pin 452 may extend in an opposite direction of the portion thereof. The second pin 452 may be adjacent to the first protruding portion 421a of the shield electrode 420. The first coupling portion 421b may be protruded from the first protruding portion 421a to contact and be coupled to the second pin 452. The first coupling portion 421b may be bent from the first protruding portion 421a to surround a portion of the second pin 452 and may be welded to the second pin 452. In other words, the shield electrode 420 may be hung on the second pin 452.

A portion of the third pin 453 may extend from the bottom 441 of the housing 440 toward a lower space of the shield electrode 420, and the remainder of the third pin 453 may extend in an opposite direction of the portion thereof. The third pin 453 may be adjacent to the first lower electrode 431. The first coupling portion 4311b may be at least a portion of the first protruding portion 4311a. The first coupling portion 4311b may be welded to a portion of the third pin 453. Alternatively, the first coupling portion 4311b may be bent as shown in the first coupling portion 411b and welded to a portion of the third pin 453. In other words, the first lower electrode 431 may be hung on the third pin 453.

A portion of the fourth pin 454 may extend from the bottom 441 of the housing 440 toward a lower space of the shield electrode 420, and the remainder of the fourth pin 454 may extend in an opposite direction of the portion thereof. The fourth pin 454 may be adjacent to the second lower electrode 435. The first coupling portion 4351b may be at least a portion of the first protruding portion 4351a. The first coupling portion 4351b may be welded to a portion of the fourth pin 454. Alternatively, the first coupling portion 4351b may be bent as shown in the first coupling portion 421b and welded to a portion of the fourth pin 454. In other words, the second lower electrode 435 may be hung on the fourth pin 454.

The first holder 461 may be disposed between the bottom 441 of the housing 440 and the electrode assembly 310. The first holder 461 may include an electrically insulating material such as plastic. For example, the first holder 461 may be PPS. At least a portion of the plurality of pins 451, 452, 453, 454, 455 may penetrate the first holder 461. For example, the third to fourth pins 453, 454 may penetrate the first holder 461. Accordingly, the first holder 461 may minimize the movement of the pin assembly 450.

The second holder 462 may be located opposite the first holder 461 with respect to the electrode assembly 310. The second holder 462 may include an electrically insulating material such as plastic. For example, the second holder 462 may be PPS. At least some of the plurality of electrodes 410, 420, 431, 433, 435 may be inserted into the second holder 462. All of the plurality of electrodes 410, 420, 431, 433, 435 may be inserted into the second holder 461. Accordingly, the second holder 462 may minimize the movement of the electrode assembly 310.

Referring again to FIG. 31, the pin assembly 450 may penetrate the bottom 441 of the housing 440, and the electrode assembly 310 may be coupled to the pin assembly 450. The electrode assembly 310 may be disposed inside the edge of the housing 440. The electrode assembly 310 may be disposed inside the circle formed by the side wall 442 of the housing 440.

The first lower electrode 431 and the second lower electrode 435 of the lower electrode assembly 430 of FIG. 31 may be spaced apart from the shield electrode 420 by a predetermined distance d downward. The shape and disposition of the lower electrode assembly 430 of FIG. 31 may correspond to the shape and disposition of the lower electrode assembly 431, 435 described above with reference to reference numeral 1001 of FIG. 10.

The lower electrode assembly 430 of the sensing module 400S of FIGS. 28 to 31 may be changed to the lower electrode assembly 431, 435 of reference numeral 1301 of FIG. 13. In this case, the number of pins of the pin assembly 450 may be four, and may be electrically connected to the upper electrode 410, the shield electrode 420, and the lower electrode assembly 431, 435. Alternatively, the pin connected to the shield electrode 420 may be omitted, in which case the number of pins of the pin assembly 450 may be three.

The lower electrode assembly 430 of the sensing module 400S of FIGS. 28 to 31 may be changed to the lower electrode assembly 431 of FIG. 4B. In this case, the number of pins of the pin assembly 450 may be three, and may be electrically connected to the upper electrode 410, the shield electrode 420, and the lower electrode assembly 431. Alternatively, the pin connected to the shield electrode 420 may be omitted, in which case the number of pins of the pin assembly 450 may be two.

The lower electrode assembly 430 of the sensing module 400S of FIGS. 28 to 31 may be changed to the lower electrode assembly 431, 433 of FIG. 5B. In this case, the number of pins of the pin assembly 450 may be four, and may be electrically connected to the upper electrode 410, the shield electrode 420, and the lower electrode assembly 431, 433. Alternatively, the pin connected to the shield electrode 420 may be omitted, in which case the number of pins of the pin assembly 450 may be three.

The lower electrode assembly 430 of the sensing module 400S of FIGS. 28 to 31 may be changed to the lower electrode assembly 431, 433, 435 of reference numeral 1002 of FIGS. 6B and 10, the lower electrode assembly 431, 433, 435 of reference numeral 1003 of FIG. 10, or the lower electrode assembly 431, 433, 435 of reference numeral 1302 of FIG. 13. In this case, the number of pins of the pin assembly 450 may be five, and may be electrically connected to the upper electrode 410, the shield electrode 420, and the lower electrode assembly 431, 433, 435. Alternatively, the pin connected to the shield electrode 420 may be omitted, in which case the number of pins of the pin assembly 450 may be four.

The lower electrode assembly 430 of the sensing module 400S of FIGS. 28 to 31 may be changed to the lower electrode assembly 431, 435 and the sub-shield electrode 425 of reference numeral 1501 of FIG. 15. In this case, the number of pins of the pin assembly 450 may be five, and may be electrically connected to the upper electrode 410, the shield electrode 420, the lower electrode assembly 431, 435, and the sub-shield electrode 425. Alternatively, the pins connected to the shield electrode 420 or the sub-shield electrode 425 may be omitted, in which case the number of pins of the pin assembly 450 may be four. Alternatively, the pins connected to the shield electrode 420 and the sub-shield electrode 425 may be omitted, in which case the number of pins of the pin assembly 450 may be three.

As described above, according to at least one embodiment of the present disclosure, the concentration of oil existing inside the compressor 1 can be accurately detected.

In addition, according to at least one embodiment of the present disclosure, the height of the oil surface can be accurately detected based on the concentration of oil existing inside the compressor 1.

In addition, according to at least one embodiment of the present disclosure, the amount of oil existing inside the compressor 1 can be accurately detected.

In addition, according to at least one embodiment of the present disclosure, a sensor 300 optimized to detect the oil concentration, the height of the oil surface and the amount of oil using a plurality of electrodes can be provided.

In addition, according to at least one embodiment of the present disclosure, by using the self-capacitance method, the detection range for the height of the oil surface may not be limited by the size of the electrodes.

In addition, according to at least one embodiment of the present disclosure, various examples of sensing modules 400S of a sensor 300 can be provided.

Referring to FIGS. 1 to 31, a sensor according to an aspect of the present disclosure may include an upper electrode extending in a horizontal direction; at least one lower electrode disposed below the upper electrode; and a shield electrode disposed between the upper electrode and the lower electrode. The shield electrode and the lower electrode may be disposed to be immersed in a fluid at least containing oil. A sensing voltage may be applied to the upper electrode and the lower electrode.

In addition, according to an aspect of the present disclosure, the sensing voltage may be applied to the shield electrode.

In addition, according to an aspect of the present disclosure, the shield electrode may be formed to extend in the horizontal direction so as to have a shape corresponding to the upper electrode, and may be disposed to correspond to the upper electrode.

In addition, according to an aspect of the present disclosure, a size of the upper electrode in the horizontal direction may be smaller than a size of the shield electrode.

In addition, according to an aspect of the present disclosure, the sensor further may include a ground electrode which is disposed below the shield electrode, and to which a ground voltage is applied.

In addition, according to an aspect of the present disclosure, the sensor may further include a capacitance detection circuit configured to detect an upper capacitance corresponding to the upper electrode and a lower capacitance corresponding to the lower electrode.

In addition, according to an aspect of the present disclosure, the sensor may further include a control circuit. The control circuit may be configured to determine a concentration of the oil, based on the lower capacitance, and determine a height of a surface of the fluid corresponding to a distance between the upper electrode and the surface of the fluid, based on the concentration of the oil and the upper capacitance.

In addition, according to an aspect of the present disclosure, a plurality of lower electrodes may be disposed below the shield electrode.

In addition, according to an aspect of the present disclosure, the capacitance detection circuit may be configured to detect a first lower capacitance corresponding to a first lower electrode and a second lower capacitance corresponding to a second lower electrode, among the plurality of lower electrodes. The control circuit may be configured to determine a first concentration of the oil corresponding to a first depth of the fluid, based on the first lower capacitance, determine a second concentration of the oil corresponding to a second depth of the fluid, based on the second lower capacitance, determine a third concentration of the oil corresponding to the surface of the fluid, based on the first concentration of the oil and the second concentration of the oil, and determine the height of the surface of the fluid, based on the third concentration of the oil and the upper capacitance.

In addition, according to an aspect of the present disclosure, the plurality of lower electrodes may be spaced apart from the shield electrode in a vertical direction by a certain distance, and disposed side by side along the horizontal direction. A width of the first lower electrode and a width of the second lower electrode with respect to the horizontal direction may be different.

In addition, according to an aspect of the present disclosure, the plurality of lower electrodes may be spaced apart from the shield electrode in a vertical direction by a certain distance, and disposed side by side along the horizontal direction. A height of a first lower electrode and a height of a second lower electrode with respect to the vertical direction may be different.

In addition, according to an aspect of the present disclosure, the sensor may further include a ground electrode which is disposed between the plurality of lower electrodes at a lower side of the shield electrode, and to which a ground voltage is applied.

In addition, according to an aspect of the present disclosure, the plurality of lower electrodes and the ground electrode may be spaced apart from the shield electrode by a certain distance in a vertical direction, and disposed side by side along the horizontal direction. A width of a first lower electrode and a width of a second lower electrode, with respect to the horizontal direction, may correspond. A distance between the first lower electrode and the ground electrode and a distance between the second lower electrode and the ground electrode, with respect to the horizontal direction, may be different.

In addition, according to an aspect of the present disclosure, the plurality of lower electrodes and the ground electrode may be spaced apart from the shield electrode by a certain distance in a vertical direction, and disposed side by side along the horizontal direction. A height of a first lower electrode and a height of a second lower electrode, with respect to the vertical direction, may correspond. A distance between the first lower electrode and the ground electrode and a distance between the second lower electrode and the ground electrode, with respect to the horizontal direction, may be different.

In addition, according to an aspect of the present disclosure, a first lower electrode of the plurality of lower electrodes may be disposed spaced apart from the shield electrode by a first distance in a vertical direction. A second lower electrode of the plurality of lower electrodes may be disposed spaced apart from the shield electrode by a second distance different from the first distance in a vertical direction.

In addition, according to an aspect of the present disclosure, the sensor may further include a ground electrode which is disposed between the plurality of lower electrodes at a lower side of the shield electrode, and to which a ground voltage is applied.

In addition, according to an aspect of the present disclosure, the ground electrode may be formed to extend along the vertical direction. The first lower electrode may be disposed on one side of the ground electrode corresponding to the horizontal direction. The second lower electrode may be disposed on the other side of the ground electrode corresponding to the horizontal direction.

In addition, according to an aspect of the present disclosure, the sensor may further include a sub-shield electrode disposed between the plurality of lower electrodes at a lower side of the shield electrode.

In addition, according to an aspect of the present disclosure, the sub-shield electrode may be formed to extend along the horizontal direction. The first lower electrode may be disposed on one side of the sub-shield electrode corresponding to the vertical direction. The second lower electrode may be disposed on the other side of the sub-shield electrode corresponding to the vertical direction.

In addition, according to an aspect of the present disclosure, the sensing voltage may be applied to the sub-shield electrode.

Since the accompanying drawings are merely for easily understanding embodiments disclosed herein, it should be understood that the technical idea disclosed herein is not limited by the accompanying drawings, and all changes, equivalents or substitutions are included in the idea and technical scope of the present disclosure.

Meanwhile, an operation method of the present disclosure can also be embodied as processor readable code on a processor-readable recording medium. The processor-readable recording medium includes all kinds of recording apparatuses storing data that can be read by a processor. Examples of the processor-readable recording medium is ROM, RAM, CD-ROM, magnetic tapes, floppy disks, optical data storage apparatuses, and, including those that are implemented in the form of carrier waves such as data transmission through the Internet. In addition, the processor-readable recording medium is dispersed in computer systems connected through a network, so that the processor-readable code can be stored and executed in a distributed fashion.

While the present disclosure has been particularly shown and described with reference to exemplary embodiments thereof, it will be understood by those of ordinary skill in the art that various changes in form and detail may be made herein without departing from the idea and scope of the present disclosure as defined by the following claims and such modifications and variations should not be understood individually from the technical idea or aspect of the present disclosure.

**Claims**

1. A sensor (300) comprising:

   an upper electrode (410) extending in a horizontal direction;
   at least one lower electrode (431, 435) disposed below the upper electrode (410), and
   a shield electrode (420) disposed between the upper electrode (410) and the lower electrode (431, 435),
   wherein the shield electrode (420) and the lower electrode (431, 435) are disposed to be immersed in a fluid at least containing oil, and
   a sensing voltage is applied to the upper electrode (410) and the lower electrode (431, 435).

2. The sensor (300) of claim 1, wherein the sensing voltage is applied to the shield electrode (420).

3. The sensor (300) of claim 1 or 2, wherein the shield electrode (420) is formed to extend in the horizontal direction so as to have a shape corresponding to the upper electrode (410), and is disposed to correspond to the upper electrode (410).

4. The sensor (300) of claim 3, wherein a size of the upper electrode (410) in the horizontal direction is smaller than a size of the shield electrode (420).

5. The sensor (300) of any one of claims 1 to 4, further comprising a ground electrode (433) which is disposed below the shield electrode (420), and to which a ground voltage is applied.

6. The sensor (300) of any one of claims 1 to 5, further comprising:

   a capacitance detection circuit (321) configured to detect an upper capacitance corresponding to the upper electrode (410) and a lower capacitance corresponding to the lower electrode (431, 435), and.
a control circuit (323) configured to:

determine a concentration of the oil, based on the lower capacitance, and
determine a height of a surface of the fluid corresponding to a distance between the upper electrode (410) and the surface of the fluid, based on the concentration of the oil and the upper capacitance.

7. The sensor (300) of claim 6, wherein a plurality of lower electrodes (431, 435) are disposed below the shield electrode (420),

wherein the capacitance detection circuit (321) is configured to detect a first lower capacitance corresponding to a first lower electrode (431) and a second lower capacitance corresponding to a second lower electrode (435), among the plurality of lower electrodes (431, 435), and
wherein the control circuit (323) is configured to:

determine a first concentration of the oil corresponding to a first depth of the fluid, based on the first lower capacitance,
determine a second concentration of the oil corresponding to a second depth of the fluid, based on the second lower capacitance,
determine a third concentration of the oil corresponding to the surface of the fluid, based on the first concentration of the oil and the second concentration of the oil, and
determine the height of the surface of the fluid, based on the third concentration of the oil and the upper capacitance.

8. The sensor (300) of any one of claims 1 to 6, wherein a plurality of lower electrodes (431, 435) are disposed below the shield electrode (420),

wherein the plurality of lower electrodes (431, 435) are spaced apart from the shield electrode (420) in a vertical direction by a certain distance, and disposed side by side along the horizontal direction, and
a width of the first lower electrode (431) and a width of the second lower electrode (435) with respect to the horizontal direction are different.

9. The sensor (300) of any one of claims 1 to 6, wherein a plurality of lower electrodes (431, 435) are disposed below the shield electrode (420),

wherein the plurality of lower electrodes (431, 435) are spaced apart from the shield electrode (420) in a vertical direction by a certain distance, and disposed side by side along the horizontal direction, and
a height of a first lower electrode (431) and a height of a second lower electrode (435) with respect to the vertical direction are different.

10. The sensor (300) of any one of claims 1 to 4, and 6, wherein a plurality of lower electrodes are disposed below the shield electrode,

wherein the sensor further comprises a ground electrode (433) which is disposed between the plurality of lower electrodes (431, 435) at a lower side of the shield electrode (420), and to which a ground voltage is applied.
wherein the plurality of lower electrodes (431, 435) and the ground electrode (433) are spaced apart from the shield electrode (420) by a certain distance in a vertical direction, and disposed side by side along the horizontal direction,
a width of a first lower electrode (431) and a width of a second lower electrode (435), with respect to the horizontal direction, correspond, and
a distance between the first lower electrode (431) and the ground electrode (433) and a distance between the second lower electrode (435) and the ground electrode (433), with respect to the horizontal direction, are different.

11. The sensor (300) of any one of claims 1 to 4, and 6, wherein a plurality of lower electrodes (431, 435) are disposed below the shield electrode (420),

wherein the sensor (300) further comprises a ground electrode (433) which is disposed between the plurality of lower electrodes (431, 435) at a lower side of the shield electrode (420), and to which a ground voltage is applied,
wherein the plurality of lower electrodes (431, 435) and the ground electrode (433) are spaced apart from the shield electrode (420) by a certain distance in a vertical direction, and disposed side by side along the horizontal direction,
a height of a first lower electrode (431) and a height of a second lower electrode (435), with respect to the vertical direction, correspond, and
a distance between the first lower electrode (431) and the ground electrode (433) and a distance between the second lower electrode (435) and the ground electrode (433), with respect to the horizontal direction, are different.

12. The sensor of any one of claims 1 to 4, and 6, wherein a plurality of lower electrodes are disposed below the shield electrode (420),

wherein a first lower electrode (431) of the plurality of lower electrodes (431, 435) is disposed spaced apart from the shield electrode (420) by a first distance in a vertical direction, and a second lower electrode (435) of the plurality of lower electrodes (431, 435) is disposed spaced apart from the shield electrode (420) by a second distance different from the first distance in a vertical direction.

13. The sensor of claim 12, further comprising a ground electrode which is disposed between the plurality of lower electrodes at a lower side of the shield electrode, and to which a ground voltage is applied,

wherein the ground electrode (433) is formed to extend along the vertical direction, the first lower electrode (431) is disposed on one side of the ground electrode (433) corresponding to the horizontal direction, and the second lower electrode (435) is disposed on the other side of the ground electrode (433) corresponding to the horizontal direction.

14. The sensor (300) of claim 12, further comprising a sub-shield electrode (425) disposed between the plurality of lower electrodes (431, 435) at a lower side of the shield electrode (420),

wherein the sub-shield electrode (425) is formed to extend along the horizontal direction, the first lower electrode (431) is disposed on one side of the sub-shield electrode (425) corresponding to the vertical direction, and the second lower electrode (435) is disposed on the other side of the sub-shield electrode (425) corresponding to the vertical direction.

15. The sensor (300) of claim 14, wherein the sensing voltage is applied to the sub-shield electrode (425).

# FIG. 1A

# FIG. 1B

# FIG. 2

260

Controller

270
Output unit

210
Communication unit

240
Fan driving unit

241
Fan

220
Sensor unit

250
Compressor driving unit

251
Compressor

Memory ~230

# FIG. 3

300

310
Electrode assembly

320

321
Capacitance detection circuit

323
Control circuit

# FIG. 4A

310

# FIG. 4B

310

# FIG. 5A

# FIG. 5B

# FIG. 6A

# FIG. 6B

# FIG. 7

# FIG. 8

<801>

<802>

<803>

# FIG. 9

<901>

<902>

<903>

# FIG. 10

420

d          d

431          w3          w5          435

+z

-x          +x

-z

<1001>

420

d          d 433          d

431          w3          w4          w5          435

+z

-x          +x

-z

<1002>

420

d          ds1          d 433          ds2          d

431          w3          w4          w5          435

+z

-x          +x

-z

<1003>

# FIG. 11

# FIG. 12

# FIG. 13

420

d1

d2

435

431

w5

w3

+z

−x +x

−z

<1301>

420

d1

d2

h4

435

431

w5

433

w3

+z

−x +x

−z

<1302>

# FIG. 14

<1401>                    <1402>

# FIG. 15

<1501>

<1502>

# FIG. 16

<1601>

<1602>

# FIG. 17

<1701>                    <1702>

# FIG. 18

# FIG. 19

# FIG. 20

# FIG. 21

# FIG. 22

# FIG. 23

# FIG. 24

# FIG. 25

# FIG. 26

# FIG. 27

# FIG. 28

# FIG. 29

# FIG. 30

# FIG. 31

Europäisches
Patentamt

European
Patent Office

Office européen
des brevets

# EUROPEAN SEARCH REPORT

**Application Number**

EP 25 16 8143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/187173 A1 (JANG YONG HEE [KR] ET AL) 30 June 2016 (2016-06-30) <br> * figures 3,11,12 * <br> * paragraphs [0106] - [0109], [0111], [0127], [0141] * | 1-15 | INV. <br> G01N27/22 <br> F01M11/12 <br> G01F23/263 <br> G01N33/28 |
| X | CN 210 603 488 U (XIE YEHUA) 22 May 2020 (2020-05-22) <br> * figure 5 * <br> * paragraphs [0002], [0004], [0055], [0056], [0057] * | 1-4,8,9, 12,14,15 | |
| A | CN 109 186 713 B (SHANGHAI INST TECH; SHANGHAI XIXI DETECTION TECH CO LTD) 28 July 2020 (2020-07-28) <br> * the whole document * | 1-15 | |
| A | EP 4 105 614 A1 (TE CONNECTIVITY GERMANY GMBH [DE]) 21 December 2022 (2022-12-21) <br> * the whole document * | 1-15 | |

TECHNICAL FIELDS
SEARCHED (IPC)

G01N
F01M
G01F

The present search report has been drawn up for all claims

| Place of search | Date of completion of the search | Examiner |
|---|---|---|
| Munich | 10 July 2025 | Kratz, Dorothee |

EPO FORM 1503 03.82 (P04C01)

# EP 4 628 883 A1

## ANNEX TO THE EUROPEAN SEARCH REPORT
## ON EUROPEAN PATENT APPLICATION NO.

EP 25 16 8143

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2025

| Patent document cited in search report | Publication date | Patent family member(s) | Publication date |
|---|---|---|---|
| US 2016187173 A1 | 30-06-2016 | BR 112017009787 A2 | 30-01-2018 |
| | | CN 105736348 A | 06-07-2016 |
| | | EP 3194786 A1 | 26-07-2017 |
| | | KR 20160081021 A | 08-07-2016 |
| | | RU 2017127052 A | 01-02-2019 |
| | | US 2016187173 A1 | 30-06-2016 |
| | | WO 2016108361 A1 | 07-07-2016 |
| CN 210603488 U | 22-05-2020 | NONE | |
| CN 109186713 B | 28-07-2020 | NONE | |
| EP 4105614 A1 | 21-12-2022 | NONE | |

EPO FORM P0459

For more details about this annex : see Official Journal of the European Patent Office, No. 12/82

57

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240045162
- KR 1020240097754
- KR 1020240123069
- KR 1020250042382